# EUROPEAN PATENT SPECIFICATION

(11) **EP 1 838 283 B1**
(45) Date of publication and mention of the grant of the patent: **31.12.2008**
(21) Application number: 05799128.3
(22) Date of filing: 03.11.2005
(51) Int. Cl.: A61K 9/127, A61P 35/00, A61P 29/00

(54) **LIPID-BASED DRUG DELIVERY SYSTEMS CONTAINING UNNATURAL PHOSPHOLIPASE A2 DEGRADABLE LIPID DERIVATIVES AND THE THERAPEUTIC USES THEREOF**
ARZNEIMITTELABGABESYSTEME AUF LIPIDBASIS MIT UNNATÜRLICHEN PHOSPHOLIPASE-A2-ABBAUBAREN LIPID-DERIVATEN UND IHRE THERAPEUTISCHE VERWENDUNG
SYSTEMES DE DELIVRANCE DE MEDICAMENTS A BASE DE LIPIDES CONTENANT DES DERIVES DE LIPIDES DEGRADABLES PHOSPHOLIPASE A2 NON NATURELS ET UTILISATIONS THERAPEUTIQUES DE CES DERNIERS

(30) Priority: 03.11.2004 DK 200401690
(43) Date of publication of application: 03.10.2007
(73) Proprietor: Liplasome Pharma A/S, 2800 Lyngby (DK)
(72) Inventor: JORGENSEN, Kent, 2880 Bagsvaerd (DK); ANDRESEN, Thomas, 2720 Vanlose (DK)
(74) Representative: Christensen, Bent
(86) International application number: PCT/DK2005/000700
(87) International publication number: WO 2006/048017

(56) References cited:
- EP-A- 1 484 332
- WO-A-01/58910
- WO-A-88/06439
- WO-A-96/39831
- JIE X ET AL: "The chemical synthesis of a series of ether phospholipids from d-mannitol and their properties" TETRAHEDRON: ASYMMETRY, ELSEVIER SCIENCE PUBLISHERS, AMSTERDAM, NL, vol. 8, no. 18, 25 September 1997 (1997-09-25), pages 3131-3142, XP004090523 ISSN: 0957-4166

## Description

### FIELD OF THE INVENTION

The invention relates to lipid-based pharmaceutical compositions used in the treatment of various disorders, e.g. cancer, infectious, and inflammatory conditions, etc., i.e. disorders and diseases associated with or resulting from increased levels of extracellular PLA₂ activity in the diseased tissue.

### BACKGROUND OF THE INVENTION

Mono-ether lyso-phospholipids and alkyl phosphocholines are known to be effective anticancer agents (see e.g. US 3,752,886 and later references). One specific example of a well-studied mono-ether alkyl phosphocholine is 1-*O*-octadecyl-2-*O*-methyl-*sn-*glycero-3-phosphocholine (ET 18-OCH₃).

Several mechanisms of the toxic action of ether-lipids towards cancer cells have been proposed involving lack of alkyl-cleavage enzymes in cancer cells. This leads to an accumulation of the ether-lipids in the cell membranes which induce membrane defects and possibly subsequent lysis. Other potential mechanisms of action include effects on intracellular protein phosphorylation and disruption of the lipid metabolism. Normal cells typically possess alkyl-cleavage enzymes, which enable them to avoid the toxic effect of ether-lipids. However, some normal cells e.g.; red blood cells, have like cancer cells no means of avoiding the disruptive effect of the ether-lipids. Accordingly, therapeutic use of ether-lipids requires an effective drug-delivery system that protects the normal cells from the toxic effects and is able to bring the ether-lipid to the diseased tissue.

US 5,985,854, US 6,077,837, US 6,136,796 and US 6,166,089 describe prodrugs with enhanced penetration into cells, which are particular useful for treating a condition or disease in a human related to supranormal intracellular enzyme activity. The prodrugs may be C-2 esters of lysophospholipids. Such drugs are designed so as to be cleaved by intracelluar phospholipase A₂.

Even in view of the above, an increasing demand for novel drug delivery systems exist, in particular drug delivery systems for targeted delivery of drug substances which are able to treat or alleviate conditions such as cancer and inflammation. Due to the fact that drugs for the treatment of cancer may be particularly harmful to tissue in general, it is of particular importance to suppress liberation of the drug substance or substances at locations other than the diseased tissue.

### BRIEF DESCRIPTION OF THE INVENTION

The present invention is directed to drug delivery systems which are particularly useful in the treatment or alleviation of diseases which are characterised by localised activity of extracelluar PLA₂ activity.

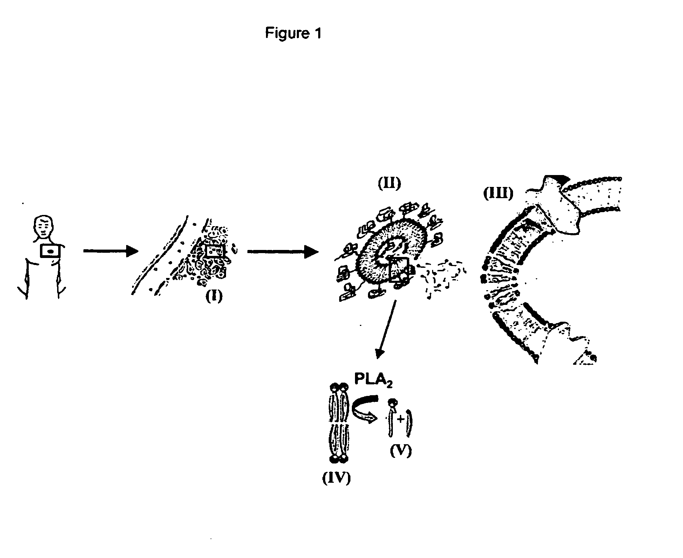

The new principle for liposomal drug targeting by extracellular PLA₂ described in this application - involves lipid-based prodrugs as illustrated in Fig 1. In this case a specific lipid-analogue compound may be incorporated into the polymer or polysaccharide chains "grafted" carrier liposome and act as a prodrug which is turned into an active drug by hydrolysis via the extracellular phospholipase. Possible examples could be certain mono-ether lipids which have been found to exhibit anti-cancer activity. If the the lipids are modified with a long fatty-acid chain that is ester linked in the C-3 position and a head group that is linked in the C-2 position and therefore as described in the present invention can be hydrolysed by extracellular PLA₂ at the target site, these modified mono-ether lipids constituting the carrier liposome will act as prodrugs. Finally it should be pointed out that certain drugs such as the anti-cancer drug adriamycin (of which doxorubicin is a derivative) themselves are known to stimulate extracellular PLA₂-activity by decreasing the calcium requirement of the enzyme.

The principle of drug targeting, release and absorption by extracellular phospholipase A2 (PLA₂) which is illustrated in Fig. 1, can be applied to a case also involving lipid-based prodrugs. In this case lipid derivatives are constituents of the carrier liposome and act as prodrugs which are turned into active drugs (e.g. ether lipids) by hydrolysis via the extracellular PLA₂ that is present in elevated concentrations in the diseased target tissue. A specific example is a prodrug of a certain mono-ether lipid which exhibits anti-cancer activity. This can be a therapeutically active compound (e.g. regulatory fatty acid derivatives) that is ester bound to the phospholipid in the C-3 position and therefore renders the lipid derivative substrate for extracellular PLA₂. If the mono-ether lipids are modified with, e.g. a ester-linked derivative in the C-3 position and therefore can be hydrolysed by extracellular PLA₂ at the target site, these lipid derivatives constituting the carrier liposome will act as prodrugs that become hydrolysed and turned into drugs by extracellular PLA₂ at the target site. In this way therapeutically active substances, e.g., monoether lipids and ester-linked derivatives will be liberated at the desired target site. Furthermore, the hydrolysis product can act as local permeability enhancers facilitating the transport of the generated anti-cancer drug into the cell. Pharmaceutical compositions containing the lipid-based system can be used therapeutically, for example, in the treatment of cancer, infectious and inflammatory conditions.

This invention provides such a delivery system in the form of lipid-based carriers, e.g. liposomes or micelles, composed of novel unnatural lipid-bilayer forming lipids such as glycerophospholipids containing an alkyl-linkage or acyl-linkage in the 1-position and an acyl-linkage or another PLA2 degradable bond, e.g. a bioisoster of a PLA2 hydrolysable ester in the C-3 position on the glycerol backbone and which have polymer or polysaccharide chains grafted thereto in the C-2 position. In addition, the carrier system may contain lipid-bilayer stabilising components, e.g. lipopolymers, glycolipids and sterols which lead to an increased vascular circulation time and as a consequence an accumulation in the diseased target tissue. When the carriers reach the target site of therapeutic action, e.g. cancer cells, PLA₂-catalyzed hydrolysis of the acyl-linkage releases the therapeutically active components, typically lyso-etherlipids and ester-linked derivatives. Contradictory to alkyl-cleavage enzymes which are nearly absent in cancer cells, extracellular PLA₂ activity is elevated in cancer tissue. In addition, extracellular PLA₂ activity is elevated in diseased regions such as inflammatory tissue.

The present invention thus provides a lipid-based drug delivery system for administration of an active drug substance selected from lysolipid derivatives, wherein the active drug substance is present in the lipid-based system in the form of a prodrug, said prodrug being a lipid derivative having (a) an aliphatic group of a length of at least 3 carbon atoms and an organic radical having at least 3 carbon atoms, and (b) a hydrophilic moiety, said prodrug furthermore being a substrate for extracellular phospholipase A2 to the extent that the organic radical can be hydrolytically cleaved off, whereas the aliphatic group remains substantially unaffected, whereby the active drug substance is liberated in the form of a lysolipid derivative, said system having included therein lipopolymers or glycolipids so as to present hydrophilic chains on the surface of the system.

The present invention also provides a lipid based drug delivery system for administration of an second drug substance, wherein the second drug substance is incorporated in the system, said system including lipid derivatives which has (a) an aliphatic group of a length of at least 3 carbon atoms and an organic radical having at least 3 carbon atoms, and (b) a hydrophilic moiety, where the lipid derivative furthermore is a substrate for extracellular phospholipase A2 to the extent that the organic radical can be hydrolytically cleaved off, so as to result in an organic acid fragment or an organic alcohol fragment and a lysolipid fragment, said system having included therein lipopolymers or glycolipids so as to present hydrophilic chains on the surface of the system.

Thus, the present invention takes advantage of the surprising finding that liposomes (and micelles) including lipid derivatives which can be specifically and only partially cleaved by extracellular phospholipases, and which at the same time includes lipopolymers or glycolipids, have the properties of circulating in the blood stream sufficiently long so as to reach target tissue where the extracellular PLA₂ activity is elevated without being recognised by the mammalian reticuloendothelial systems and without penetrating cell walls, whereby the lipid derivatives of the liposomes are specifically cleaved by extracellular PLA₂ so as to liberate therapeutically active ingredients at the desired location.

The present invention also provides a class of novel lipid derivatives which are particularly useful as constituents of the drug delivery systems described herein.

### DESCRIPTION OF THE DRAWINGS

Fig. 1. Schematic illustration of the lipid-based drug-targeting and drug-triggering principle involving accumulation of the liposomal drug carriers in porous diseased tissue and subsequent release of drug and transport across the target membrane via extracellular PLA₂ activity.
   (I) Pathological tissue with leaky capillaries
   (II) Prodrug and drug carrying liposome
   (III) Target cell and cell membrane
   (IV) Novel and unnatural lipid analogs e.g. prodrug (lipid), proenhancer (lipid), proactivator (lipid)
   (V) Drugs (lysolipid and fatty acid derivatives), enhancers (lysolipid + fatty acid), PLA2 activators (lysolipid + fatty acid)

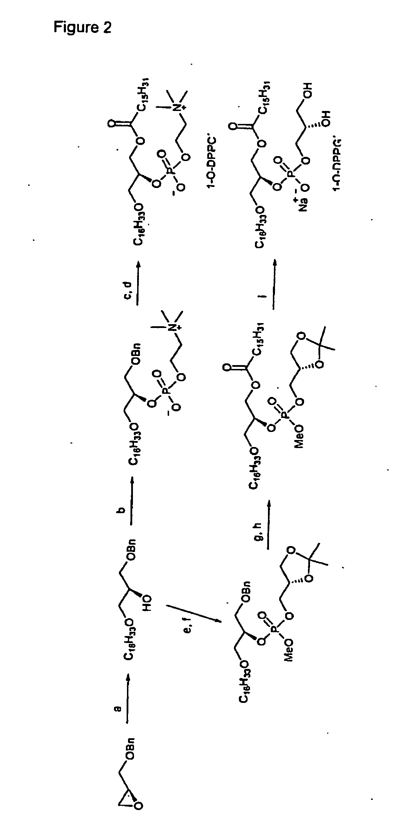

Fig. 2. Synthesis of the novel phospholipid analogs 1-O-DPPC' and 1-O-DPPG' with the phosphate in the C-2 position from (S)-O-benzyl glycidol. (a) C₁₆H₃₃OH, NaH, DMF/THF (71%); (b) i. POCl₃, Et₃N, DCM, ii. Pyridine, Choline tosylate (65%); (c) H₂, Pd/C, MeOH (99%); (d) C₁₅H₃₁COOH, DMAP, Et₃N, DCM (83%); (e) (i-Pr)₂NP(OMe)Cl, TMP, DCM; (f) i. (R)-isopropylidene glycerol, Phenyl-1H-tetrazole, DCM, ii. t-BuOOH (67% over 2 steps); (g) H₂, Pd/C, MeOH (99%); (h) Palmitic acid, DMAP, DCC, DCM (92%); (i) i. CH₃CN, isopropanol, Me₃N, DCM, ii. HCl, MeOH, DCM, H₂O, iii. NaHCO₃, DCM (70%).

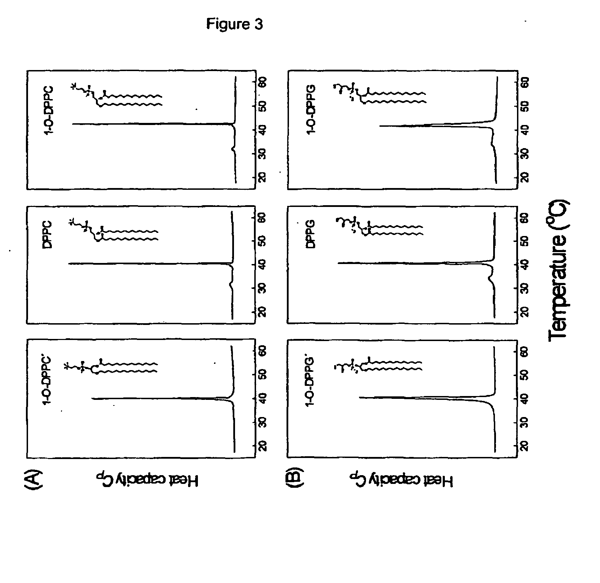

Fig. 3. Heat capacity, C_{P}, obtained using differential scanning calorimetry at a scan rate of 20°C/h for 0.15 mM multilamellar liposomes: (A) 1-O-DPPC' (left), DPPC (middle), and 1-O-DPPC (right). (B) 1-O-DPPG' (left), DPPG (middle) and 1-O-DPPG (right).

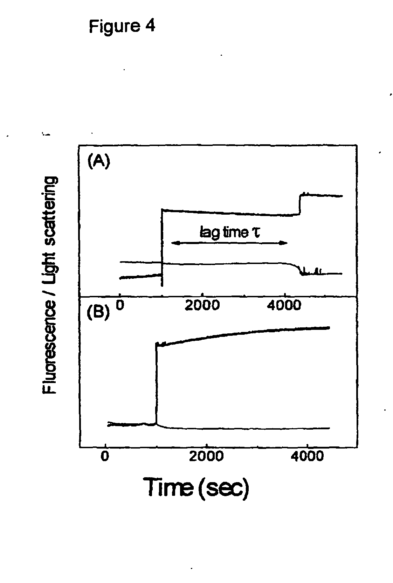

Fig. 4. Characteristic reaction time profiles at 40°C for PLA2 hydrolysis of unilamellar. (A) 0.15 mM 1-O-DPPC' liposomes incubated with 150 nM PLA2 (A. piscivorus piscivorus). (B) 0.15 mM 1-O-DPPG' liposomes incubated with human secretory PLA2 from 20 µL freshly prepared human tear fluid [42] (B). The PLA2 hydrolysis reaction is monitored by intrinsic fluorescence (solid line) from the enzyme and 90° static light scattering (dashed lines) from the lipid suspension. After adding PLA2 to the equilibrated liposome suspension a characteristic lag time, τ, follows before a sudden increase in the catalytic activity takes place (A). This lag time is observed for the neutrally charged 1-O-DPPC' liposomes but not for the negatively charged 1-O-DPPG' liposomes (B) where the enzyme becomes active immediately after addition.

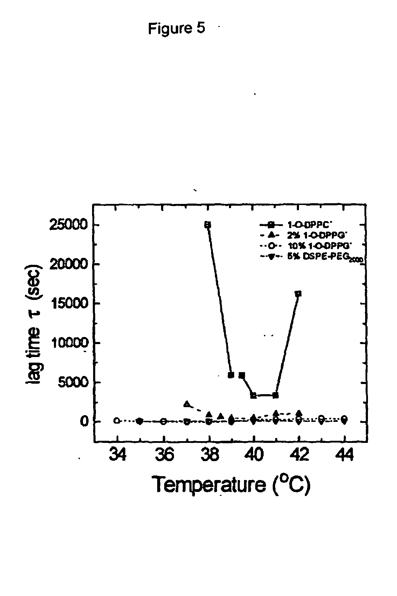

Fig. 5. PLA2 (A. piscivorus piscivorus) lag time, τ, as a function of temperature for the hydrolysis of 1-O-DPPC' unilamellar liposomes incorporated with 0 mol% (■), 2 mol% (▲), and 10 mol% (○) 1-O-DPPG' lipids, and 5 mol% (▼) DSPE-PEG₂₀₀₀ polymer lipids. The concentration of the liposomes was 0.15 mM and the concentration of PLA2 was 150 nM.

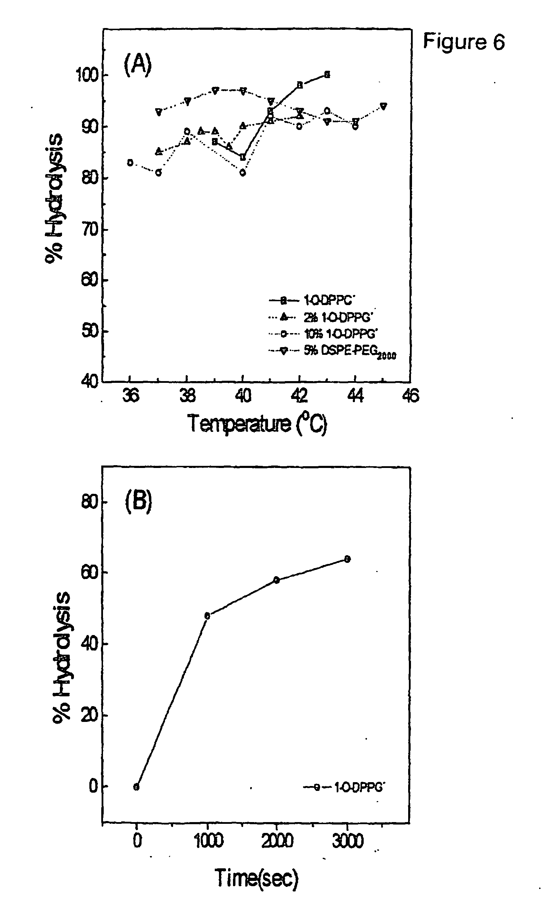

Fig. 6. (A) PLA2 (A. piscivorus piscivorus) hydrolysis of 1-O-DPPC' liposomes containing 0 mol% (■), 2 mol% (▲), and 10 mol% (○) 1-O-DPPG' lipids, and 5 mol% (▼) DSPE-PEG₂₀₀₀ polymer lipids determined by HPLC [28]. The percent lipid hydrolysis is determined 1000 s after the unset of the burst. The concentration of the liposomes was 0.15 mM and the concentration of PLA2 was 150 nM. (B) Hydrolysis of 1-O-DPPG' unilamellar liposomes determined by HPLC [28]. The PLA2 catalyzed lipid hydrolysis is determined as a function of time after addition of 20 µL freshly prepared human tear fluid [42] to 0.15 mM 1-O-DPPG' liposomes.

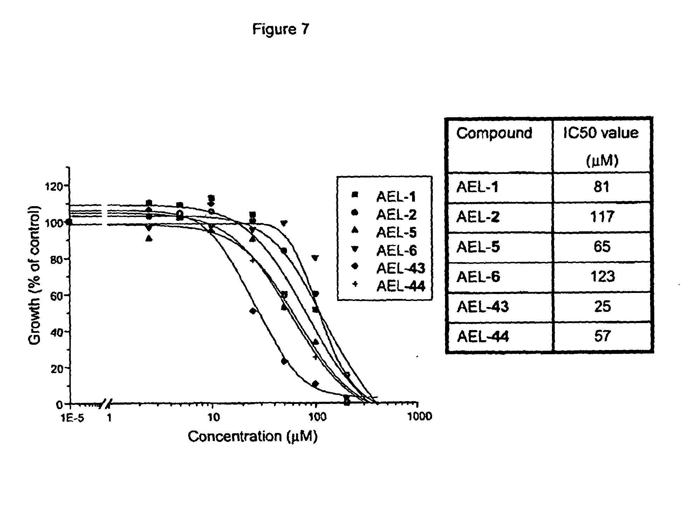

Fig. 7. Graph showing the cytotoxic activity of AEL 43 and 44. Their activity was compared with AEL-1,2,5 and 6 in the HT-29 colon cancer cell line. Cells were exposed to the compounds for 72 hours and the cytotoxic activity was evaluated by the MTT method (Carmichael et al. Cancer Res 1987;47:936).

AEL-1, 1-*O*-Hexadecyl-2-lyso-*sn*-glycero-3-phosphocholine; AEL-2, 1-*O-*Hexadecyl-2-lyso-*sn*-glycero-3-phospho-(*S*)-glycerol; AEL-5, 1-*O*-Octadecyl-2-lyso-*sn*-glycero-3-phosphocholine; AEL-6, 1-*O*-Octadecyl-2-lyso-*sn*-glycero-3-phospho-(*S*)-glycerol; AEL-43, (*R*)-1-*O*-Hexadecyl-3-lyso-glycero-2-phosphocholine; AEL-44, (*R*)-1-*O*-Hexadecyl-3-lyso-glycero-2-phospho-(*S*)-glycerol.

**Table 1. Differential scanning calorimetry data showing the melting enthalpy, ΔH, half width, T_{1/2}, and peak position, Tₘ, of the pre- and main phase transitions.**

| | Pre-transition | | | Main-transition | | |
|---|---|---|---|---|---|---|
| | Δ*H* (kcal/mol) | *T*_{*1*/}*₂* (°C) | *Tₘ* (°C) | Δ*Hₘ* (kcal/mol) | *T*_{*1*/}*₂* (°C) | *Tₘ* (°C) |
| 1-O-DPPC' | | - | - | 12.8 | 0.25 | 40.1 |
| DPPC | 1.2 | 1.9 | 31.9 | 8.7 | 0.21 | 40.5 |
| 1-O-DPPC | 0.84 | 1.23 | 32.0 | 8.5 | 0.24 | 42.6 |
| 1-O-DPPC' | - | - | - | 11.3 | 0.71 | 40.6 |
| DPPG | 1.31 | 2.2 | 34.3 | 9.8 | 0.41 | 40.6 |
| 1-O-DPPG | 0.58 | 2.7 | 34.1 | 9.1 | 0.86 | 41.8 |

### DETAILED DESCRIPTION OF THE INVENTION

One of the important features of the present invention is the realisation that certain lipid derivatives will be cleaved by extracellular PLA₂ in a well-defined manner in extracellular locations of mammalian diseased tissue. It has been found that extracellular PLA₂ is capable of cleaving monoether/monoester lipid derivatives so as to produce monoether lysolipid derivatives which as such, or in combination with other active compounds, will exhibit a therapeutic effect.

### Lipid derivatives

Thus, the drug delivery systems (liposomes or micelles) of the present invention relies on lipid derivatives having (a) an aliphatic group of a length of at least 3 carbon atoms and an organic radical having at least 3 carbon atoms, and (b) a hydrophilic moiety, said prodrug furthermore being a substrate for extracellular phospholipase A2 to the extent that the organic radical can be hydrolytically cleaved off, whereas the aliphatic group remains substantially unaffected, whereby the active drug substance is liberated in the form of a lysolipid derivative, said system having included therein lipopolymers or glycolipids so as to present hydrophilic chains on the surface of the system.

Although the terms "lipid" and "lysolipid" (in the context of phospholipids) will be well-known terms for the person skilled in the art, it should be emphasised that, within the present description and claims, the term "lipid" is intended to mean triesters of glycerol of the following formula: 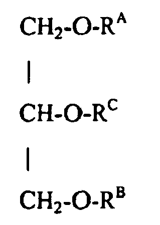 wherein R^{A} and R^{B} are fatty acid moieties (C₉₋₃₀-alkyl/alkylene/alkyldiene/- alkyltriene/alkyltetraene-C(=O)-) and R^{C} is a phosphatidic acid (PO₂-OH) or a derivative of phosphatidic acid or a bioisoster of phosphatidic acid. Thus, the groups R^{A} and R^{B} are linked to the glycerol backbone via ester bonds.

The term "lysolipid" is intended to mean a lipid where the R^{B} fatty acid group is absent (e.g. hydrolytically cleaved off), i.e. a glycerol derivative of the formula above where R^{B} is hydrogen, but where the other substituents are substantially unaffected. Conversion of a lipid to a lysolipid can take place under the action of an enzyme, specifically under the action of cellular as well as extracellular PLA₂.

The terms "lipid derivative" and "lysolipid derivative" are intended to cover possible derivatives of the above possible compounds within the groups "lipid" and "lysolipid", respectively. Examples of biologically active lipid derivatives and lysolipid derivatives are given in Houlihan, et al., Med. Res. Rev., 15, 3, 157-223. Thus, as will be evident, the extension "derivative" should be understood in the broadest sense.

Within the present application, lipid derivatives and lysolipids should however fulfil certain functional criteria (see above) and/or structural requirements. It is particularly relevant to note that the suitable lipid derivatives are those which have (a) an aliphatic group of a length of at least 3, preferably at least 9, carbon atoms and an organic radical having at least 3 carbon atoms, and (b) a hydrophilic moiety. It will be evident that the aliphatic group and the organic radical will correspond to the two fatty acid moieties in a normal lipid and that the hydrophilic moiety will correspond to the phosphate part of a (phospho)lipid or a bioisoster thereof.

Thus, as the general idea behind the present invention is to exploit the increased level of extracellular PLA₂ activity in localised areas of the body of a mammal, in particular diseased tissue, the lipid derivatives which can be utilised within the present invention should be substrates for extracellular PLA₂, i.e. the lipid derivatives should be able to undergo hydrolytic, enzymatic cleavage of the organic radical corresponding to a fatty acid in the 3-position in a lipid. Extracellular PLA₂ is known to belong to the enzyme class (EC) 3.1.1.4. Thus by reference to (extracellular) PLA₂ should be understood all extracellular enzymes of this class, e.g. lipases, which can induce hydrolytic cleavage of the organic radical corresponding to the fatty acid in the 2-position in a lipid. One particular advantage of the lipid based drug delivery system (as liposomes and micelles) is that extracellular PLA₂ activity is significantly increased towards organised substrates as compared to monomeric substrates.

In view of the requirement to hydrolysability by extracellular PLA₂, it is clear that the organic radical (e.g. aliphatic group) is preferably linked via an ester functionality which can be cleaved by extracellular PLA₂, preferably so that the group which is cleaved off is a carboxylic acid.

Furthermore, it is an important feature of the present invention that the aliphatic group (the group corresponding to the fatty acid in the 1-position in a lipid) of the lipid derivative, i.e. the lysolipid derivative after cleavage by extracellular PLA₂, is substantially unaffected by the action of extracellular PLA₂. By "substantially unaffected" is meant that the integrity of the aliphatic group is preserved and that less than 1 mol%, preferably less than 0.1 mol%, of the aliphatic group (the aliphatic group in the 1-position) is cleaved under the action of extracellular PLA₂.

One preferred class of lipid derivatives for incorporation in the drug delivery systems of the invention can be represented by the following formula: 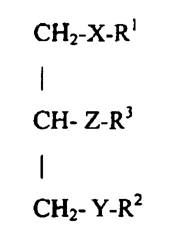 wherein
X and Z independently are selected from OC(O), O, CH₂, NH, NMe, S, S(O), OS(O), S(O)₂, OS(O)₂, OP(O)₂, OP(O)₂O, OAs(O)₂ and OAs(O)₂O; preferably from O, NH, NMe and CH₂, in particular O and CH₂;
Y is selected from OC(O), OC(O)O, OC(O)N, OC(S), SC(O), SC(S), CH₂C(O)O, NC(O)O, Y then being connected to R² via either the oxygen, sulphur, nitrogen or carbonyl carbon atom, preferably via the carbonyl carbon atom;
R¹ is an aliphatic group of the formula Y¹Y²;
R² is an organic radical having at least 2 carbon atoms, such as an aliphatic group having a length of at least 2, preferably at least 9, carbon atoms, preferably a group of the formula Y¹Y²;
where Y¹ is -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈-(CH₂)ₙ₉, and the sum of nl+2n2+n3+2n4+n5+2n6+n7+2n8+n9 is an integer of from 2 to 29; n 1 is zero or an integer of from 1 to 29, n3 is zero or an integer of from 1 to 20, n5 is zero or an integer of from 1 to 17, n7 is zero or an integer of from 1 to 14, and n9 is zero or an integer of from 1 to 11; and each of n2, n4, n6 and n8 is independently zero or 1; and Y² is CH₃, OH, SH, S(O)₂OH, P(O)₂OH, OP(O)₂OH, NH₂ or CO₂H; where each Y¹-Y² independently may be substituted with halogens and aliphatic substituents, but preferably Y¹-Y² is unsubstituted,
R³ is selected from phosphatidic acid (PO₂-OH), derivatives of phosphatidic acid and bioisosters to phosphatic acid, e.g. P(O)O, P(O)₂CH₂, S(O)O, S(O)CH₂, C(O)O, C(O)N, C(S)O, P(S)O₂, S(O)₂CH₂ and derivatives thereof (among others phosphatidic acid derivatives to which a hydrophilic polymer or polysaccharide is covalently attached).

As mentioned above, preferred embodiments imply that Y is -OC(O)- where Y is connected to R² via the carboxyl atom. The most preferred embodiments imply that X and Z are O and that Y is -OC(O)- where Y is connected to R² via the carboxyl atom. This means that the lipid derivative is a 1-monoether-2-monoester-phospholipid type compound.

Another preferred group of lipid derivatives is the one where the group X is S.

In one embodiment, R¹ and R² are aliphatic groups of the formula Y¹ Y² where Y² is CH₃, OH, SH, S(O)₂OH, P(O)₂OH, OP(O)₂OH, NH₂ or CO₂H, but preferably CH₃, and where Y¹ is -(CH₂)ₙ₁(CH=CH)ₙ₂(CH₂)ₙ₃(CH=CH)ₙ₄(CH₂)ₙ₅(CH=CH)ₙ₆(CH₂)ₙ₇-(CH=CH)ₙ₈(CH₂)ₙ₉; the sum of n1+2n2+n3+2n4+n5+2n6+n7+ 2n8+n9 is an integer of from 2 to 29; that is, the aliphatic group, Y¹Y², is from 2-29 carbon atoms in length. n1 is equal to zero or is an integer of from 1 to 23; n3 is equal to zero or is an integer of from 1 to 20; n5 is equal to zero or is an integer of from 1 to 17; n7 is equal to zero or is an integer of from 1 to 14; n9 is equal to zero or is an integer of from 1 to 11; and each of n2, n4, n6 and 8 is independently equal to zero or 1.

Although the aliphatic groups may be unsaturated and even substituted with halogens (flouro, chloro, bromo, iodo) and C₁₋₁₀-groups (i.e. yielding branched aliphatic groups), the aliphatic groups as R¹ and R² are in one embodiment preferably saturated as well as unbranched, that is, they preferably have no double bonds between adjacent carbon atoms, each of n2, n4, n6 and n8 then being equal to zero. Accordingly, Y¹ is preferably (CH₂)ₙ₁. More preferably (in this embodiment), R¹ and R² are each independently (CH₂)ₙ₁CH₃, and most preferably, (CH₂)₁₇CH₃ or (CH₂)₁₅CH₃. In alternative embodiments, the groups can have one or more double bonds, that is, they can be unsaturated, and one or more of n2, n4, n6 and n8 can be equal to 1. For example, when the unsaturated hydrocarbon has one double bond, n2 is equal to 1, n4, n6 and n8 are each equal to zero and Y¹ is (CH₂)ₙ₁ CH=CH(CH₂)ₙ₃. n 1 is equal to zero or is an integer of from 1 to 21, and n3 is also zero or is an integer of from 1 to 20, at least one of n1 or n3 not being equal to zero.

In one particular embodiment, the lipid derivatives are those which are monoether lipids where X and Z are O, R¹ and R² are independently selected from alkyl groups, (CH₂)ₙCH₃, where n is 11, 12, 13, 14, 15, 16, 17, 18, 19, 20, 21, 22, 23, 24, 25, 26, 27, 28, or 29, preferably 14, 15 or 16, in particular 14; Y is -OC(O)-, Y then being connected to R² via the carbonyl carbon atom.

With respect to the hydrophilic moiety (often known as the "head group") which corresponds to R³, it is believed that a wide variety of groups corresponding to phosphatidic acid (PO₂-OH), derivatives of phosphatidic acid and bioisosters to phosphatic acid and derivatives thereof can be used. As will be evident, the crucial requirement to R³ is that the groups should allow for enzymatic cleavage of the R² group (e.g. R²-C(=O) or R²-OH) by extracellular PLA₂. "Bioisosters to phosphatidic acid and derivatives thereof" indeed implies that such groups - as phosphatidic acid - should allow for enzymatic cleavage by extracellular PLA₂.

R³ is typically selected from phosphatidic acid (PO₂-OH), phosphatidylcholine (PO₂-O-CH₂CH₂N(CH₃)₃), phosphatidylethanolamine (PO₂-O-CH₂CH₂NH₂), N-methyl-phosphatidylethanolamine (PO₂-O-CH₂CH₂NHCH₃), , phosphatidylserine, phosphatidylinositol, and phosphatidylglycerol (PO₂-O-CH₂CHOHCH₂OH). Other possible derivatives of phosphatidic acid are those where dicarboxylic acids, such as glutaric, sebacic, succinic and tartaric acids, are coupled to the terminal nitrogen of phosphatidylethanolamines, phosphatidylserine, phosphatidylinositol, etc.

In the particular embodiment where a fraction of the lipid derivative also is a lipopolymer or glycolipid, a hydrophilic polymer or polysaccharide is typically covalently attached to the phosphatidyl part of the lipid derivative. Another particular lipid derivative include an acyl chain attached to the head group of the lipids,

Hydrophilic polymers which suitable can be incorporated in the lipid derivatives of the invention so as to form lipopolymers are those which are readily water-soluble, can be covalently attached to a vesicle-forming lipid, and which are tolerated *in vivo* without toxic effects (i.e. are biocompatible). Suitable polymers include polyethylene glycol (PEG), polylactic (also termed polylactide), polyglycolic acid (also termed polyglycolide), a polylactic-polyglycolic acid copolymer, polyvinyl alcohol, polyvinylpyrrolidone, polymethoxazoline, polyethyloxazoline, polyhydroxypropyl methacrylamide, polymethacrylamide, polydimethylacrylamide, and derivatised celluloses such as hydroxymethylcellulose or hydroxyethylcellulose.

Preferred polymers are those having a molecular weight of from about 100 daltons up to about 10,000 daltons, and more preferably from about 300 daltons to about 5,000 daltons. In a particularly preferred embodiment, the polymer is polyethyleneglycol having a molecular weight of from about 100 to about 5,000 daltons, and more preferably having a molecular weight of from about 300 to about 5,000 daltons. In a particularly preferred embodiment, the polymer is polyethyleneglycol of 750 daltons (PEG(750)). Polymers may also be defined by the number of monomers therein; a preferred embodiment of the present invention utilises polymers of at least about three monomers, such PEG polymers consisting of three monomers (approximately 150 daltons).

When the glycolipid or lipopolymer is represented by a fraction of the lipid derivative, such a lipid derivative (lipid derivative with a polymer or polysaccharide chain) typically constitutes 1-80 mol%, such as 2-50 mol% or 3-25 mol% of the total dehydrated lipid-based system. For micellular compositions, however, the fraction may be even higher, such as from 1-100 mol%, such as 10-100 mol%, of the total dehydrated lipid-based system.

Preferred polymers to be covalently linked to the phosphatidyl part (e.g. via the terminal nitrogen of phosphatidylethanolamine) are polyethylene glycol (PEG), polyactide, polyglycolic acid, polyactide-polyglycolic acid copolymer, and polyvinyl alcohol.

One highly interesting aspect of the present invention is the possibility of modifying the pharmaceutical effect of the lipid derivative by modifying the group R². It should be understood that R² should be an organic radical having at least 2 carbon atoms) (such as an aliphatic group having a certain length (at least 2, preferably 9, carbon atoms)), a high degree of variability is possible, e.g. R² need not necessarily to be a long chain residue, but may represent more complex structures.

Generally, it is believed that R² may either be rather inert for the environment in which it can be liberated by extracellular PLA₂ or that R² may play an active pharmaceutical role, typically as an auxiliary drug substance or as an efficiency modifier for the lysolipid derivative and/or any other (second) drug substances present in the environment.

In some embodiments, the R¹ and R² groups will be long chain residues, e.g. a fatty acid residue (the fatty acid will include a carbonyl from the group Y). This has been described in detail above. Interesting examples of auxiliary drug substances as R² within this subgroups are polyunsaturated acids, e.g. oleate, linoleic, linonleic, as well as derivatives of arachidonoyl (including the carbonyl from Y), e.g. prostaglandins such as prostaglandin E₁, as arachidonic acid derivatives are know regulators of hormone action including the action of prostaglandins, thromboxanes, and leukotrines. Examples of efficiency modifiers as R² are those which enhance the permeability of the target cell membrane as well as enhances the activity of extracellular PLA₂ or the active drug substance or any second drug substances. Examples hereof are short chain (C₈₋₁₂) fatty acids.

However, it is also envisaged that other groups might be useful as the organic radical R², e.g. vitamin D derivatives, steroid derivatives, retinoic acid (including all-trans-retinoic acid, all-cis-retinoic acid, 9-cis-retinoic acid, 13-cis-retinoic acid), cholecalciferol and tocopherol analogues, pharmacologically active carboxylic acids such as branched-chain aliphatic carboxylic acids (e.g. valproic acid and those described in WO 99/02485), salicylic acids (e.g. acetylsalicylic acid), steroidal carboxylic acids (e.g. lysergic and isolysergic acids), monoheterocyclic carboxylic acids (e.g. nicotinic acid) and polyheterocyclic carboxylic acids (e.g. penicillins and cephalosporins), diclofenac, indomethacin, ibuprofen, naproxen, 6-methoxy-2-naphthylacetic acid.

It should be understood that the various examples of possible R² groups are referred to by the name of a discrete species, rather than the name of the radical. Furthermore, it should be understood that the possible examples may include the carbonyl group or oxy group of the bond via which the organic radical is linked to the lipid skeleton (corresponding to "Y" in the formula above). This will of course be appreciated by the person skilled in the art.

Even though it has not specifically been indicated in the general formula for the suitable examples of lipid derivatives to be used within the present invention, it should be understood that the glycol moiety of the lipid derivatives may be substituted, e.g. in order to modify the cleavage rate by extracellular PLA₂ or simply in order to modify the properties of the liposomes comprising the lipid derivatives.

Some of the above defined lipid derivatives may already be known, but is believed that some subgroups thereof are uniquely novel compounds.

A particular group of novel compounds is lipid derivatives of the following formula: 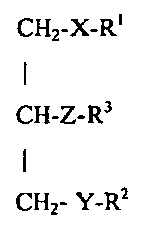 wherein
X and Z independently are selected from OC(O), O, CH₂, NH, NMe, S, S(O), OS(O), S(O)₂, OS(O)₂, OP(O)₂, OP(O)₂O, OAs(O)₂ and OAs(O)₂O; preferably from O, NH, NMe and CH₂, in particular O and CH₂;
Y is selected from OC(O), OC(O)O, OC(O)N, OC(S), SC(O), SC(S), CH₂C(O)O, NC(O)O, Y then being connected to R² via either the oxygen, sulphur, nitrogen or carbonyl carbon atom, preferably via the carbonyl carbon atom;
R¹ is an aliphatic group of the formula Y¹Y²;
R² is an organic radical having at least 2 carbon atoms;
where Y¹ is -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈-(CH₂)ₙ₉, and the sum of n1+2n2+n3+2n4+n5+2n6+n7+2n8+n9 is an integer of from 2 to 29; n 1 is zero or an integer of from 1 to 29, n3 is zero or an integer of from 1 to 20, n5 is zero or an integer of from 1 to 17, n7 is zero or an integer of from 1 to 14, and n9 is zero or an integer of from 1 to 11; and each of n2, n4, n6 and n8 is independently zero or 1; and Y² is CH₃, OH, SH, S(O)₂OH, P(O)₂OH, OP(O)₂OH, NH, or CO₂H; where each Y¹-Y² independently may be substituted with halogen or C₁₋₄-alkyl, but preferably Y¹-Y² is unsubstituted,
R³ is selected from derivatives of phosphatidic acid to which a hydrophilic polymer or polysaccharide is attached. The hydrophilic polymer or polysaccharide is typically and preferably selected from polyethylene glycol, poly(lactic acid), poly(glycolic acid), poly(lactic acid)-poly(glycolic acid) copolymers, polyvinyl alcohol, polyvinylpyrrolidone, polymethoxazoline, polyethyloxazoline, polyhydroxypropyl methacrylamide, polymethacrylamide, polydimethylacrylamide, and derivatised celluloses, in particular from polyethylene glycol, poly(lactic acid), poly(glycolic acid), poly(lactic acid)-poly(glycolic acid) copolymers, and polyvinyl alcohol.

A particular subgroups are those wherein X and Z are O, R¹ and R² are independently selected from alkyl groups, (CH₂)ₙCH₃, where n is 11, 12, 13, 14, 15, 16, 17, 18, 19, 20, 21, 22, 23, 24, 25, 26, 27, 28, or 29, preferably 14 or 16; Y is -OC(O)-, Y then being connected to R² via the carbonyl carbon atom.

A specific group of compounds are polyethyleneoxide-1-O-palmityl-*sn*-2-palmitoyl-phosphatidyl ethanolamine, DPPE-PEG, and polyethyleneoxide-1-O-stearyl-*sn*-2-stearoylphosphatidylethanolamine, DSPE-PEG, with PEG molecular weight from 100 to 10000 Daltons, in particular from 300-5000 Daltons. fraction

Furthermore, the present invention provides a pharmaceutical composition comprising a pharmaceutically acceptable carrier and a lipid derivative as defined above. Preferably, the lipid derivative in such a composition is dispersed in the form of a liposome (see below).

Also, the present invention relates to such lipid derivatives for use as a medicament, preferably present in a pharmaceutical composition, and to the use of a lipid derivative as defined above for the preparation of a medicament for the treatment of diseases or conditions associated with a localised increase in extracellular phospholipase A2 activity in mammalian tissue. Such diseases or conditions are typically selected from cancer, e.g. a brain, breast, lung, colon or ovarian cancer, or a leukemia, lymphoma, sarcoma, carcinoma, and inflammatory conditions . The present compositions and uses are especially applicable in the instances where the increase in extracellular PLA₂ activity is at least 25% compared to the normal level of activity in the tissue in question, the tissue being that of a mammal, in particular a human.

### Lipid derivatives as prodrugs

As described above, the present invention provides a lipid-based drug delivery system for administration of an active drug substance selected from lysolipid derivatives, wherein the active drug substance is present in the lipid-based system in the form of a prodrug, said prodrug being a lipid derivative having (a) an aliphatic group of a length of at least 3 carbon atoms and an organic radical having at least 3 carbon atoms, and (b) a hydrophilic moiety, said prodrug furthermore being a substrate for extracellular phospholipase A2 to the extent that the organic radical can be hydrolytically cleaved off, whereas the aliphatic group remains substantially unaffected, whereby the active drug substance is liberated in the form of a lysolipid derivative, said system having included therein lipopolymers or glycolipids so as to present hydrophilic chains on the surface of the system.

By the term "active drug substance" is meant any chemical entity which will provide a prophylactic or therapeutic effect in the body of a mammal, in particular a human. Thus, the present invention mainly relates to the therapeutic field.

The term "prodrug" should be understood in the normal sense, namely as a drug which is masked or protected with the purpose of being converted (typically by cleavage, but also by in vivo chemical conversion) to the intended drug substance. The person skilled in the art will recognise the scope of the term "prodrug".

The active drug substance is selected from lysolipid derivatives, and as it will be understood from the present description with claims, the lysolipid derivatives relevant within the present invention will have a therapeutic effect - at least - in connection with diseases and conditions where a local area of the body of the mammal has a level of extracellular PLA₂ activity which can liberate the lysolipid derivative.

As will be understood from the present description with claims, the lipid derivative will often constitute the prodrug referred to above and the lysolipid derivative will thereby constitute the active drug substance often a monoether lysolipid derivative. It should however be understood that this does not exclude the possibility of including other drug substances, referred to as second drug substances, in the drug delivery systems of the invention, neither does it exclude that the organic radical which can be hydrolytically cleaved by the action of extracellular PLA₂ can have a certain pharmaceutical effect (e.g. as an auxiliary drug substance or an efficiency modifier as described elsewhere herein). Furthermore, the pharmaceutical effect of the "active drug substance", i.e. the lysolipid derivative, need not the be the most predominant when a second drug substance is included, actually the effect of the second drug substance might very well be the most predominant as will become apparent in the other main embodiment (see "Lipid derivative liposomes as drug delivery systems", below).

The active drug substance (lysolipid derivative) release from the prodrug (lipid derivative) is believed to take place as illustrated in the following example:

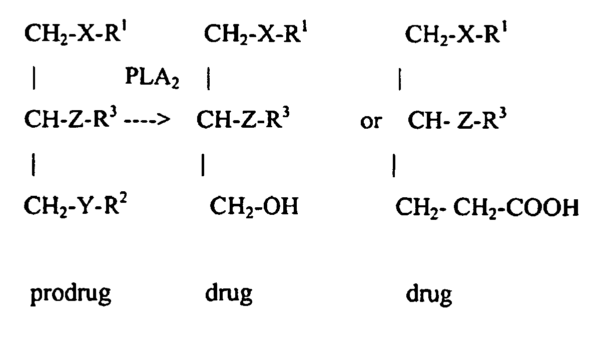

Furthermore, the substituent R² may constitute an auxiliary drug substance or an efficiency modifier for the active drug substance and will simultaneously be released under the action of extracellular PLA₂:

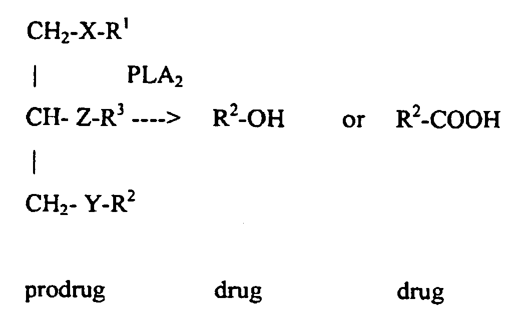

It has been described above under the definition of R² how the group R² can have various independent or synergistic effects in association with the active drug substance, e.g. as an auxiliary drug substance or an efficiency modifier, e.g. permeability or cell lysis modifier. It should be borne in mind that the groups corresponding to R² (e.g. R²-OH or R²-COOH) might have a pharmaceutical effect which is predominant in relation the effect of the lysolipid derivative (active drug substance).

### Lipid derivatives formulated as liposomes

The term "lipid-based drug delivery system" should encompass macromolecular structures which as the main constituent include lipid or lipid derivatives. Suitable examples hereof are liposomes and micelles. It is presently believed that liposomes offer the broadest scope of applications and those have been described most detailed in the following. Although liposomes currently are believed to be the preferred lipid-based system, micellular systems are also believed to offer interesting embodiments within the present invention.

In one important variant which advantageously can be combined with the embodiments described herein, the lipid derivative (e.g. the prodrug) is included in liposomes either as the only constituent or - which is more common - in combination with other constituents (other lipids, sterols, etc.). Thus, the lipid-based systems described herein are preferably in the form of liposomes, wherein the liposomes are build up of layers comprising the lipid derivative (e.g. a prodrug).

"Liposomes" are known as self-assembling structures comprising one or more lipid bilayers, each of which surrounds an aqueous compartment and comprises two opposing monolayers of amphipathic lipid molecules. Amphipathic lipids (i.e. lipid derivatives) comprise a polar (hydrophilic) headgroup region (corresponding to the substituent R³ in the lipid derivatives) covalently linked to one or two non-polar (hydrophobic) aliphatic groups (corresponding to R¹ and R² in the lipid derivatives). Energetically unfavourable contacts between the hydrophobic groups and the aqueous medium are generally believed to induce lipid molecules to rearrange such that the polar headgroups are oriented towards the aqueous medium while the hydrophobic groups reorient towards the interior of the bilayer. An energetically stable structure is formed in which the hydrophobic groups are effectively shielded from coming into contact with the aqueous medium.

Liposomes can have a single lipid bilayer (unilamellar liposomes, "ULVs"), or multiple lipid bilayers (multilamellar liposomes, "MLVs"), and can be made by a variety of methods (for a review, see, for example, Deamer and Uster, Liposomes, Marcel Dekker, N.Y., 1983, 27-52). These methods include Bangham's methods for making multilamellar liposomes (MLVs); Lenk's, Fountain's and Cullis' methods for making MLVs with substantially equal interlamellar solute distribution (see, e.g., US 4,522,803, US 4,588,578, US 5,030,453, US 5,169,637 and US 4,975,282); and Papahadjopoulos et al.'s reverse-phase evaporation method (US 4,235,871) for preparing oligolamellar liposomes. ULVs can be produced from MLVs by such methods as sonication (see Papahadjopoulos et al., Biochem. Biophys. Acta, 135, 624 (1968)) or extrusion (US 5,008,050 and US 5,059,421). The liposome of this invention can be produced by the methods of any of these disclosures, the contents of which are incorporated herein by reference.

Various methodologies, such as sonication, homogenisation, French Press application and milling can be used to prepare liposomes of a smaller size from larger liposomes. Extrusion (see US 5,008,050) can be used to size reduce liposomes, that is to produce liposomes having a predetermined mean size by forcing the liposomes, under pressure, through filter pores of a defined, selected size. Tangential flow filtration (see WO 89/08846), can also be used to regularise the size of liposomes, that is, to produce liposomes having a population of liposomes having less size heterogeneity, and a more homogeneous, defined size distribution. The contents of these documents are incorporated herein by reference. Liposome sizes can also be determined by a number of techniques, such as quasi-electric light scattering, and with equipment, e.g., Nicomp^{®} particle sizers, well within the possession of ordinarily skilled artisans.

It is quite interesting to note that the lipid derivatives of the present invention can constitute the major part of a lipid-based system even if this system is a liposome system. This fact resides in the structural (but not functional) similarity between the lipid derivatives of the present invention and lipids. Thus, it is believed that the lipid derivatives for the present invention can be the sole constituent of liposomes, i.e. up to 100 mol% of the total dehydrated liposomes can be constituted by the lipid derivatives. This is in contrast to the known mono-ether lysolipids like ET-18-OCH3, which can only constitute a minor part of the liposomes.

Typically, as will be described in detail below, liposomes advantageously include other constituents which may or may not have a pharmaceutical effect, but which will render the liposome structure more stable (or alternatively more unstable) or will protect the liposomes against clearance and will thereby increase the circulation time thereby improving the overall efficiency of a pharmaceutical including the liposome.

This being said, it is believed that the particular lipid derivatives will typically constitute from 5-100 mol%, such as 50-100 mol%, preferably from 75-100 mol%, in particular 90-100 mol%, based on the total dehydrated liposome.

The liposomes can be unilamellar or multilamellar. Some preferred liposomes are unilamellar and have diameters of less than about 400 nm, more preferably, from greater than about 40 nm to less than about 400 nm.

The liposomes are typically - as known in the art - prepared by a method comprising the steps of: (a) dissolving the lipid derivative in an organic solvent; (b) removing the organic solvent from the lipid derivative solution of step (a); and (c) hydrating the product of step (b) with an aqueous solvent so as to form liposomes.

The method may further comprise a step of adding an second drug substance (see below) to the organic solvent of step (a) or the aqueous phase of step (c).

Subsequently, the method may comprise a step of extruding the liposomes produced in step (c) through a filter to produce liposomes of a certain size, e.g. 100 nm.

Lipid-based particulate systems, i.e. liposomes as well as micelles; of sizes covering a broad range may be prepared according to the above-mentioned techniques. Depending on the route of administration, suitable sizes for pharmaceutical applications will normally be in the range of 20-10,000 nm, in particular in the range of 30-1000 nm. Sizes in the range of 50-200 nm are normally preferred because liposomes in this size range are generally believed to circulate longer in the vascular system of mammals than do larger liposomes which are more quickly recognised by the mammals' reticuloendothelial systems ("RES"), and hence, more quickly cleared from the circulation. Longer vascular circulation can enhance therapeutic efficacy by allowing more liposomes to reach their intended site of actions, e.g., tumours or inflammations.

It is believed that for a drug delivery system as defined in the embodiments herein, which is adapted to be administered via intraveneous and intramuscular injection, the liposomes should preferably have a mean particle size of about 100 nm. Thus, the particle size should generally be in the range of 40-400 nm.

Furthermore, for a drug delivery system adapted to be administered via subcutaneous injection, the liposomes should preferably have a mean particle size from 100 to 5000 nm, and the liposomes can then be uni- or multilayered.

One of the advantages by including the lipid derivatives in liposomes is that the liposome structure, in particular when stabilised as described in the following, will have a much longer vascular circulation time that the lipid derivatives as discrete compounds. Furthermore, the lipid derivatives will become more or less inert or even "invisible" when "packed" in liposomes in which lipopolymers or glycolipids are included. This means than any potential disadvantageous effect, e.g. hemolytic effect, can be suppressed.

The liposomes should preferably act as inert constituents until they reach the area of interest, e.g. cancerous, infected or inflammatorily diseased areas or tissue. As will be described in the following, liposomes may include a number of other constituents. In particular, a drug delivery system according to the invention may further contain a component which controls the release of any second drug substance, extracellular PLA₂ activity controlling agents or permeability enhancer, e.g. short chain lipids and lipopolymers/glycolipids.

Two very important groups of compounds to be included in liposomes as modifiers are the stabilising compound lipopolymers and glycolipids, such as lipopolymers (e.g. polyethyleneoxide-dipalmitoylphosphatidyl ethanolamine, DPPE-PEG, polyethyleneoxide-distearoylphosphatidylethanolamine, DSPE-PEG) with PEG molecular weight from 100 to 10000 Daltons. It has been shown that lipopolymers function as stabilisers for the liposome, i.e. lipopolymer increases the circulation time, and - which is highly interesting in the present context, as activators for extracellular PLA₂. The stabilising effect will be described in the following.

Liposome outer surfaces are believed to become coated with serum proteins, such as opsonins, in mammals' circulatory systems. Without intending in any way to be limited by any particular theory, it is believed that liposome clearance can be inhibited by modifying the outer surface of liposomes such that binding of serum proteins thereto is generally inhibited. Effective surface modification, that is, alterations to the outer surfaces of liposomes which result in inhibition of opsonisation and RES uptake, is believed to be accomplished by incorporating into liposomal bilayers lipids whose polar headgroups have been derivatised by attachment thereto of a chemical moiety which can inhibit the binding of serum proteins to liposomes such that the pharmacokinetic behaviour of the liposomes in the circulatory systems of mammals is altered and the activity of extracellular PLA₂ is enhanced as described for the lipopolymers above.

Liposome preparations have been devised which avoid rapid RES uptake and which thus have an increased half-life in the bloodstream. STEALTH^{®} liposomes (Liposome Technology Inc., Menlo Park, Calif.) include polyethyleneglycol (PEG)-grafted lipids at about 5 mol% of the total dehydrated liposome . The presence of polymers on the exterior liposome surface decreases the uptake of liposomes by the organs of the RES. The liposome membranes can be constructed so as to resist the disruptive effects of the surfactant contained therein. For example, a liposome membrane which contains as constituents lipids derivatised with a hydrophilic (i.e., water-soluble) polymer normally has increased stability. The polymer component of the lipid bilayer protects the liposome from uptake by the RES, and thus the circulation time of the liposomes in the bloodstream is extended.

Hydrophilic polymers suitable for use in lipopolymers are those which are readily water-soluble, can be covalently attached to a vesicle-forming lipid, and which are tolerated in vivo without toxic effects (i.e., are biocompatible). Suitable polymers include polyethylene glycol (PEG), polylactic (also termed polylactide), polyglycolic acid (also termed polyglycolide), a polylactic-polyglycolic acid copolymer, and polyvinyl alcohol. Preferred polymers are those having a molecular weight of from about 100 or 120 daltons up to about 5,000 or 10,000 daltons, and more preferably from about 300 daltons to about 5,000 daltons. In a particularly preferred embodiment, the polymer is polyethyleneglycol having a molecular weight of from about 100 to about 5,000 daltons, and more preferably having a molecular weight of from about 300 to about 5,000 daltons. In a particularly preferred embodiment, the polymer is polyethyleneglycol of 750 daltons (PEG(750)). Polymers may also be defined by the number of monomers therein; a preferred embodiment of the present invention utilises polymers of at least about three monomers, such PEG polymers consisting of three monomers (approximately 150 daltons). Other hydrophilic polymers which may be suitable for use in the present invention include polyvinylpyrrolidone, polymethoxazoline, polyethyloxazoline, polyhydroxypropyl methacrylamide, polymethacrylamide, polydimethylacrylamide, and derivatised celluloses such as hydroxymethylcellulose or hydroxyethylcellulose.

Glycolipids are lipids to which a hydrophilic polysaccharide chain is covalently attached. It will be appreciated that glycolipids can be utilised like lipopolymers although the lipopolymers currently presents the most promising results.

It is generally believed that the content of lipopolymer advantageously will be in the range of 1-50 mol%, such as 2-25%, in particular 2-15 mol%, based on the total dehydrated liposome.
The liposomes' bi- or multilayers may also contain other constituents such as other lipids, sterolic compounds, polymer-ceramides as stabilisers and targeting compounds, etc.

The liposomes comprising lipid derivatives may (in principle) exclusively consist of the lipid derivatives. However, in order to modify the liposomes, "other lipids" may be included as well. Other lipids are selected for their ability to adapt compatible packing conformations with the lipid derivative components of the bilayer such that the all the lipid constituents are tightly packed, and release of the lipid derivatives from the bilayer is inhibited. Lipid-based factors contributing to compatible packing conformations are well known to ordinarily skilled artisans and include, without limitation, acyl chain length and degree of unsaturation, as well as the headgroup size and charge. Accordingly, suitable other lipids, including various phosphatidylethanolamines ("PE's") such as egg phosphatidylethanolamine ("EPE") or dioleoyl phosphatidylethanolamine ("DOPE"), can be selected by ordinarily skilled artisans without undue experimentation. Lipids may be modified in various way, e.g. by headgroup derivatisation with dicarboxylic acids, such as glutaric, sebacic, succinic and tartaric acids, preferably the dicarboxylic acid is glutaric acid ("GA"). Accordingly, suitable headgroup-derivatised lipids include phosphatidylethanolamine-dicarboxylic acids such as dipalmitoyl phosphatidylethanolamine-glutaric acid ("DPPE-GA"), palmitoyloleoyl phosphatidylethanolamine-glutaric acid ("POPE-GA") and dioleoyl phosphatidylethanolamine-glutaric acid ("DOPE-GA"). Most preferably, the derivatised lipid is DOPE-GA.

The total content of "other lipids" will typically be in the range of 0-30 mol%, in particular 1-10 mol%, based on the total dehydrated liposome.

Sterolic compound included in the liposome may generally affects the fluidity of lipid bilayers. Accordingly, sterol interactions with surrounding hydrocarbon groups generally inhibit emigration of these groups from the bilayer. An examples of a sterolic compound (sterol) to be included in the liposome is cholesterol, but a variety of other sterolic compounds are possible. It is generally believed that the content of sterolic compound, if present, will be in the range of 0-25 mol%, in particular 0-10 mol%, such as 0-5 mol%, based on the total dehydrated liposome.

Polymer-ceramides are stabilisers improving the vascular circulation time. Examples are polyethylene glycol derivatives of ceramides (PEG-ceramides), in particular those where the molecular weight of the polyethylene glycol is from 100 to 5000. It is generally believed that the content of polymer-ceramides, will be in the range of 0-30 mol%, in particular 0-10 mol%, based on the total dehydrated liposome.

Still other ingredients may constitute 0-2 mol%, in particular 0-1 mol%, based on the total dehydrated liposome.

According to an embodiment of the present invention, the lipid bilayer of a liposome contains lipids derivatised with polyethylene glycol (PEG), such that the PEG chains extend from the inner surface of the lipid bilayer into the interior space encapsulated by the liposome, and extend from the exterior of the lipid bilayer into the surrounding environment (see e.g. US 5,882,679 and Fig. 1).

A variety of coupling methods for preparing a vesicle-forming lipid derivatised with a biocompatible, hydrophilic polymer such as polyethylene glycol are known in the art (see, e.g., US 5,213,804; US 5,013,556).

The derivatised lipid components of liposomes according to the present invention may additionally include a labile lipid-polymer linkage, such as a peptide, ester, or disulfide linkage, which can be cleaved under selective patophysiological conditions, such as in the presence of overexpressed peptidase or esterase enzymes at diseased sites or reducing agents. Use of such linkages to couple polymers to phospholipids allows the attainment of high blood levels of such liposomes for several hours after administration, followed by cleavage of the reversible linkages and removal of the polymer from the exterior liposome bilayer. The polymer-less liposomes are then subject to rapid uptake by the RES system (see, e.g., US 5,356,633).

Additionally, liposomes according to the present invention may contain non-polymer molecules bound to the exterior of the liposome, such as haptens, enzymes, antibodies or antibody fragments, cytokines and hormones (see, e.g., US 5,527,528), and other small proteins, polypeptides, single sugar polysaccharide moieties, or non-protein molecules which confer a particular enzymatic or surface recognition feature to the liposome. See published PCT application WO 94/21235. Surface molecules which preferentially target the liposome to specific organs or cell types are referred to herein as "targeting molecules" and include, for example, antibodies and sugar moieties, e.g. gangliosides or those based on mannose and galactose, which target the liposome to specific cells bearing specific antigens (receptors for sugar moieties). Techniques for coupling surface molecules to liposomes are known in the art (see, e.g., US 4,762,915).

The liposome can be dehydrated, stored and then reconstituted such that a substantial portion of its internal contents is retained. Liposomal dehydration generally requires use of a hydrophilic drying protectant such as a disaccharide sugar at both the inside and outside surfaces of the liposome bilayers (see US 4,880,635). This hydrophilic compound is generally believed to prevent the rearrangement of the lipids in the liposome, so that the size and contents are maintained during the drying procedure and through subsequent rehydration. Appropriate qualities for such drying protectants are that they are strong hydrogen bond acceptors, and possess stereochemical features that preserve the intramolecular spacing of the liposome bilayer components. Alternatively, the drying protectant can be omitted if the liposome preparation is not frozen prior to dehydration, and sufficient water remains in the preparation subsequent to dehydration.

### Lipid derivative liposomes as drug carrier systems

As mentioned above, the liposomes including the lipid derivatives of the present invention may also include second drug substances. In a particular embodiment, the lipid-based drug delivery system described above is in the form of liposomes wherein a second drug substance is incorporated. It should be understood that second drug substances may comprise pharmaceutically active ingredients which may have an individual or synergistic pharmaceutical effect in combination with the lipid derivative and lysolipid derivatives. The term "second" does not necessarily imply that the pharmaceutical effect of the second drug substance is inferior in relation to that of, e.g., the active drug substance derived from the prodrug, but is merely used to differentiate between the two groups of substances.

This being said, the present invention also provides a drug delivery system which is in the form of liposomes, and wherein a second drug substance is incorporated.

A possible "second drug substance" is any compound or composition of matter that can be administered to mammals, preferably humans. Such agents can have biological activity in mammals. Second drug substances which may be associated with liposomes include, but are not limited to: antiviral agents such as acyclovir, zidovudine and the interferons; antibacterial agents such as aminoglycosides, cephalosporins and tetracyclines; antifungal agents such as polyene antibiotics, imidazoles and triazoles; antimetabolic agents such as folic acid, and purine and pyrimidine analogs; antineoplastic agents such as the anthracycline antibiotics and plant alkaloids; sterols such as cholesterol; carbohydrates, e.g., sugars and starches; amino acids, peptides, proteins such as cell receptor proteins, immunoglobulins, enzymes, hormones, neurotransmitters and glycoproteins; dyes; radiolabels such as radioisotopes and radioisotope-labeled compounds; radiopaque compounds; fluorescent compounds; mydriatic compounds; bronchodilators; local anesthetics; and the like.

Liposomal second drug substance formulations enhance the therapeutic index of the second drug substances by reducing the toxicity of the drug. Liposomes can also reduce the rate at which a second drug substance is cleared from the vascular circulation of mammals. Accordingly, liposomal formulation of second drug substance can mean that less of the drug need be administered to achieve the desired effect.

Liposomes can be loaded with one or more second drug substances by solubilising the drug in the lipid or aqueous phase used to prepare the liposomes. Alternatively, ionisable second drug substances can be loaded into liposomes by first forming the liposomes, establishing an electrochemical potential, e.g., by way of a pH gradient, across the outermost liposomal bilayer, and then adding the ionisable second drug substance to the aqueous medium external to the liposome (see, e.g., US 5,077,056 and WO 86/01102).

Methods of preparing lipophilic drug derivatives which are suitable for liposome or micelle formulation are known in the art (see e.g., US 5,534,499 and US 6,118,011 describing covalent attachment of therapeutic agents to a fatty acid chain of a phospholipid). A micellar formulation of taxol is described in Alkan-Onkyuksel et al., Pharmaceutical Research, 11:206 (1994).

Accordingly, the second drug substance may be any of a wide variety of known and possible pharmaceutically active ingredients, but is preferably a therapeutically and/or prophylactically active substance. Due to the mechanism involved in the degradation of the liposomes of the present invention, it is preferred that the second drug substance is one relating to diseases and/or conditions associated with a localised increase in extracellular PLA₂ activity.

Particularly interesting second drug substances are selected from (i) antitumour agents such as anthracyline derivatives, cisplatin, paclitaxel, 5-fluoruracil, exisulind, cis-retinoic acid, suldinac sulfide, vincristine, interleukins, oligonucleotides, peptides, proteins and cytokines (ii) antibiotics and antifungals, and (iii) antiinflammatory agents such as steroids and non-steroids. In particular the steroids can also have a stabilising effect on the liposomes.

Also active agents like peptides and protein derivatives like interferons, interleukins and oligonucleotides can be incorporated into the PLA2 degradable lipid-based carrier.

The cytotoxic effects of a broad range of anticancer agents are likely to improve when encapsulated in the carriers of this invention. Furthermore, it is expected that the hydrolysis products, i.e. monoether lysolipids and/or ester-linked lysolipid derivatives, act in turn with the released fatty acid derivatives as absorption enhancers for drug permeation across the target membranes when the carriers locally are broken down in the diseased tissue.

It is envisaged that the second drug substance will be distributed in the liposomes according to their hydrophilicity, i.e. hydrophilic second drug substances will tend to be present in the cavity of the liposomes and hydrophobic second drug substances will tend to be present in the hydrophobic bilayer. Method for incorporation of second drug substances are know in the art as has been made clear above.

It should be understood from the above, that the lipid derivatives may - as prodrugs or discrete constituents - posses a pharmaceutical activity. However, in a particular embodiment, the present invention furthermore relates to a lipid based drug delivery system for administration of an second drug substance, wherein the second drug substance is incorporated in the system (e.g. where the second drug substance is encapsulated in the interior of the liposome or in the membrane part of the liposome or the core region of micelle), said system including lipid derivatives which has (a) an aliphatic group of a length of at least 3 carbon atoms and an organic radical having at least 3 carbon atoms, and (b) a hydrophilic moiety, where the lipid derivative furthermore is a substrate for extracellular phospholipase A2 to the extent that the organic radical can be hydrolytically cleaved off, whereas the aliphatic group remains substantially unaffected, so as to result in an organic acid fragment or an organic alcohol fragment and a lysolipid fragment, said system having included therein lipopolymers or glycolipids so as to present hydrophilic chains on the surface of the system.

As mentioned above for the system according to the other embodiment, the organic radical which can be hydrolytically cleaved off, may be an auxiliary drug substance or an efficiency modifier for the second drug substance. It should be understood that the lipid derivative is a lipid derivative as defined further above. Typically, the lipid derivative constitutes 5-100 mol%, such as 50-100 mol%, of the total dehydrated (liposome) system.

As should be understood from the above, the present invention also provides a pharmaceutical composition comprising a pharmaceutically acceptable carrier and any of the lipid-based drug delivery systems described above. The composition will be described in detail below.

The present invention also relates to the use of any of the lipid-based drug delivery systems described herein as a medicament, and to the use of any of the lipid-based drug delivery systems described herein for the preparation of a medicament for the treatment of diseases or conditions associated with a localised increase in extracellular phospholipase A2 activity in mammalian tissue. Such diseases or conditions are typically selected from cancer, e.g. a brain, breast, lung, colon or ovarian cancer, or a leukemia, lymphoma, sarcoma, carcinoma and inflammatory conditions. Also included is the prophylactic use. The present compositions and uses are especially applicable in the instances the increase in extracellular PLA₂ activity is at least 25% compared to the normal level of activity in the tissue in question, the tissue being that of a mammal, in particular a human.

It should be mentioned that the novel and unnatural lipid analogs can be administred in a non-particulate form as free agents leading to an increased intracellular cell uptake rendering them favourable substrates for overexpressed intracellular PLA2 in the diseased target cells.

### Pharmaceutical preparations and therapeutic uses

Also provided herewith is a pharmaceutical composition comprising a pharmaceutically acceptable carrier and the lipid derivative, e.g. as a liposome, of this invention.

"Pharmaceutically acceptable carriers" as used herein are those media generally acceptable for use in connection with the administration of lipids and liposomes, including liposomal drug formulations, to mammals, including humans. Pharmaceutically acceptable carriers are generally formulated according to a number of factors well within the purview of the ordinarily skilled artisan to determine and account for, including without limitation: the particular active drug substance and/or second drug substance used, the liposome preparation, its concentration, stability and intended bioavailability; the disease, disorder or condition being treated with the liposomal composition; the subject, its age, size and general condition; and the composition's intended route of administration, e.g., nasal, oral, ophthalmic, subcutaneous, intramammary, intraperitoneal, intravenous, or intramuscular. Typical pharmaceutically acceptable carriers used in parenteral drug administration include, for example, D5W, an aqueous solution containing 5% weight by volume of dextrose, and physiological saline. Pharmaceutically acceptable carriers can contain additional ingredients, for example those which enhance the stability of the active ingredients included, such as preservatives and anti-oxidants.
The liposome or lipid derivative is typically formulated in a dispersion medium, e.g. a pharmaceutically acceptable aqueous medium.

An amount of the composition comprising an anticancer effective amount of the lipid derivative, typically from about 0.1 to about 1000 mg of the lipid derivative per kg of the mammal's body, is administered, preferably intravenously. For the purposes of this invention, "anticancer effective amounts" of liposomal lipid derivatives are amounts effective to inhibit, ameliorate, lessen or prevent establishment, growth, metastasis or invasion of one or more cancers in mammals to which the lipid derivatives have been administered. Anticancer effective amounts are generally chosen in accordance with a number of factors, e.g., the age, size and general condition of the subject, the cancer being treated and the intended route of administration, and determined by a variety of means, for example, dose ranging trials, well known to, and readily practised by, ordinarily skilled artisans given the teachings of this invention. Antineoplastic effective amounts of the liposomal drugs/prodrugs of this invention are about the same as such amounts of free, nonliposomal, drugs/prodrugs, e.g., from about 0.1 mg of the lipid derivative per kg of body weight of the mammal being treated to about 1000 mg per kg.

Preferably, the liposome administered is a unilamellar liposome having an average diameter of from about 50 nm to about 200 nm. The anti-cancer treatment method can include administration of one or more second drug substances in addition to the liposomal drug, these additional agents being included in the same liposome as the lipid derivative. The second drug substances, which can be entrapped in liposomes' internal compartments or sequestered in their lipid bilayers, are preferably, but not necessarily, anticancer agents.

The pharmaceutical composition is preferably administered parenterally by injection, infusion or implantation (intravenous, intramuscular, intraarticular, subcutaneous or the like) in dosage forms, formulations or e.g. suitable delivery devices or implants containing conventional, non-toxic pharmaceutically acceptable carriers and adjuvants.

The formulation and preparation of such compositions is well-known to those skilled in the art of pharmaceutical formulation. Specific formulations can be found in the textbook entitled "Remington's Pharmaceutical Sciences".

Thus, the pharmaceutical compositions according to the invention may comprise the active drug substances in the form of a sterile injection. To prepare such a composition, the suitable active drug substances are dispersed in a parenterally acceptable liquid vehicle which conveniently may comprise suspending, solubilising, stabilising, pH-adjusting agents and/or dispersing agents. Among acceptable vehicles that may be employed are water, water adjusted to a suitable pH by addition of an appropriate amount of hydrochloric acid, sodium hydroxide or a suitable buffer, 1,3-butanediol, Ringer's solution and isotonic sodium chloride solution. The aqueous formulation may also contain one or more preservatives, for example, methyl, ethyl or n-propyl p-hydroxybenzoate.

Where treatment of a tumour or neoplasm is desired, effective delivery of a liposome-encapsulated drug via the bloodstream requires that the liposome be able to penetrate the continuous (but "leaky") endothelial layer and underlying basement membrane surrounding the vessels supplying blood to a tumour. Liposomes of smaller sizes have been found to be more effective at extravasation into tumours through the endothelial cell barrier and underlying basement membrane which separates a capillary from tumour cells.

As used herein, "solid tumours" are those growing in an anatomical site other than the bloodstream (in contrast to blood-borne tumours such as leukemias). Solid tumours require the formation of small blood vessels and capillaries to nourish the growing tumour tissue.

In accordance with the present invention, the anti-tumour or anti-neoplastic agent of choice is entrapped within a liposome according to the present invention; the liposomes are formulated to be of a size known to penetrate the endothelial and basement membrane barriers. The resulting liposomal formulation can be administered parenterally to a subject in need of such treatment, preferably by intravenous administration. Tumours characterised by an acute increase in permeability of the vasculature in the region of tumour growth are particularly suited for treatment by the present methods. The liposomes will eventually degrade due to lipase action at the tumour site, or can be made permeable by, for example, thermal or ultrasonic radiation. The drug is then released in a bioavailable, transportable solubilised form. Furthermore, a small elevation in temperature as often seen in diseased tissue may further increase the stimulation of extracellular PLA₂.

Where site-specific treatment of inflammation is desired, effective liposome delivery of an drug requires that the liposome have a long blood half-life, and be capable of penetrating the continuous endothelial cell layer and underlying basement membrane surrounding blood vessels adjacent to the site of inflammation. Liposomes of smaller sizes have been found to be more effective at extravasation through the endothelial cell barrier and into associated inflamed regions. However, the limited drug-carrying capacity of conventional small liposome preparations has limited their effectiveness for such purposes.

In accordance with the present invention, the anti-inflammatory agent of choice is entrapped within a liposome according to the present invention; the liposomes are formulated to be of a size known to penetrate the endothelial and basement membrane barriers. The resulting liposomal formulation can be administered parenterally to a subject in need of such treatment, preferably by intravenous administration. Inflamed regions characterised by an acute increase in permeability of the vasculature in the region of inflammation are particularly suited for treatment by the present methods.

It is known that the activity of extracellular PLA₂ is abnormally high in areas of the mammalian body diseased by cancer, inflammation, etc. The present invention have provided a way of exploiting this fact, and it is believed that the extracellular PLA₂ activity should be at least 25% higher in the diseases area of the body (determined in the extracellular environment) compared with a comparative normal area. It is however envisaged that the level of extracellular PLA₂ activity often is much higher, e.g. at least 100%, e.g. at least 200% such as at least 400%. This means that treatment of a mammal in need of a treatment with the purpose of cure or relief, can be conducted with only minimal influence on tissue having a "normal" level of extracellular PLA₂ activity. This is extremely relevant in particular with the treatment of cancer where rather harsh drug (second drug substances) are often needed.

Residing in the realisations behind the present invention, the invention thus provides to a method for selectively drug targeting to diseased areas, such as areas comprising neoplastic cells, e.g., areas within the mammalian body, preferably a human, having a extracellular phospholipase A2 (extracellular PLA₂) activity which is at least 25% higher compared to the normal activity in said areas, by administering to the mammal in need thereof an efficient amount of a drug delivery system defined herein.

Provided is also a method of treating of a mammal afflicted with a cancer, e.g., a brain, breast, lung, colon or ovarian cancer, or a leukemia, lymphoma, sarcoma, carcinoma, which comprises administering a pharmaceutical composition of this invention to the mammal. It is believed that the lipid derivatives and/or second drug substance in liposome form is selectively cytotoxic to tumour cells.

### Targeting of diseased sites in the skin with lipid-based carriers composed of the novel and unnatural lipids

The novel targeted liposomal prodrug and drug delivery systems are useful in the treatment or alleviation of disorders in the skin that are associated with or resulting from increased levels of extracellular PLA₂. In particular, the lipid-based prodrug and drug delivery systems are useful for the administration of an active drug substance selected from ether-lysolipid and/or fatty acid derivatives of a lipid prodrug being a substrate for extracellular PLA₂.

Degradation of the flexible liposomal carrier by PLA₂ leads to the release of lysolipid and/or fatty acid derivatives, which are designed to be effective in the treatment of various types of skin diseases such as cancer and inflammation. In addition, the novel prodrug liposomal carriers can be used for targeted delivery of conventional drugs to diseased regions of the skin that is associated with an elevated level of PLA₂.

There are a large number of skin diseases that are either skin-specific, e.g. cancer, inflammation, psoriasis, and eczemas, or are manifestations of general diseases. A number of these diseases confined to the skin or mucous membranes could be cured or alleviated by a local topical application of pharmaceutical formulations provided that the application results in an efficient uptake of the active component. Many formulations are applied topically to the skin for therapeutic or preventive purposes. However, the skin forms one of the body's most effective barriers to foreign substances and owing to skin impermeability, many therapeutic agents must be applied *per os* or parenterally even though the skin is the target organ.

Liposomal formulations have been the focus of extensive investigation as the mode of skin delivery for many drugs. There is growing evidence that for topical administration, a new generation of flexible liposomes present several advantages over other formulations. Such advantages include reduced side-effects related to high systemic absorption of the administered drug, increased accumulation of the administered drug at the desired target, and the ability to administer a wide variety of drugs, both hydrophilic and hydrophobic, into the skin.

The major physico-chemical barrier of the skin is localized in the outermost comified layer of the skin, the stratum corneum. The pores in the stratum corneum and the underlaying structures are normally so narrow, that the skin only allows passage of entities smaller than 400 Dalton. However, it has been shown that ultraflexible liposomes are able to pass pores in the skin smaller than 30 nm (Cevc et al. (Biochem Biophys Acta (1998) 1368, p 201-215, US Patent 6.180.353).

As important as getting the substance into the skin is the issue of ensuring that predominantly the relevant cells are exposed to the pharmaceutical active components. The novel targeted prodrug system addresses both the important issue of ensuring that predominantly the relevant cells are exposed to the drugs, and the fact that only very flexible liposomes that contains "edge active substances" are able to pass through the stratum corneum and penetrate deep into diseased regions of the skin with elevated levels of PLA₂. It is important to realize that the prodrug liposomes are not intended for application of drugs into the blood via the skin.

It has been found that the activity of extracellular phospholipase A₂ (PLA₂) is increased in a number of inflammatory diseases of the skin most notably psoreasis and eczema (Forster et al. (1985) Br J Dermatol 112:135-47; Forster et al. (1983) Br J Dermatol 108:103-5). It has furthermore been found that extracellular PLA₂ is capable of cleaving the prodrug lipid derivatives so as to produce ether-lysolipid derivatives, which alone or in combination with other active compounds, will exhibit an effect. Thus the new prodrug liposomes ensure that the pharmaceutical active ether-lysolipids can be delivered specifically at the target cells in the skin using elevated activity of PLA₂ as a site-specific trigger mechanism. In addition, specific fatty acid derivatives, which are turned into active drugs by PLA₂ hydrolysis may be linked to the C-3 position of the lipid-based carrier composed of the novel unnatural lipids. Possible examples include polysaturated fatty acids and other lipophilic groups such as vitamin D derivatives, steroid derivatives, retinoyl derivatives, and tocopherol analogues that can be ester bound to the C-3 position and therefore renders the double prodrug lipid a substrate for PLA₂. Furthermore, conventional drug substances can be incorporated and transported specifically to the diseased site by the novel lipid-based carriers.

Thus the new carriers liposomes offer a solution to both the issue of penetration of intact skin and the issue of specific targeting of cells, tissues or part of tissues in the skin that are characterized by an increased level of PLA₂.

### Imaging of diseased tissue with contrast carriers composed of the novel and unnatural lipids

The novel lipid-based carrier systems are useful in the targeted diagnosis of diseases such as cancer, infection, and inflammation, which are characterized by localized and elevated activity of extracellular PLA₂. The increased PLA₂ activity combined with the observation that microparticulates accumulate in the diseased tissue via extravasation through leaky capillaries provide the basis for using contrast agent microcarriers for enhanced imaging.

Thus, the novel diagnostic system takes advantage of the fact that liposomes (and micelles), which can be specifically degraded by extracellular PLA₂ also can be designed to circulate in the blood stream sufficiently long to reach the target tissue where the PLA₂ activity is elevated. At the diseased site the lipid derivatives of the liposomes are cleaved by PLA₂ so as to liberate the image enhancing agents.

Diagnostic imaging is widely used in medicine. It requires that an appropriate intensity of signal from an area of interest is achieved in order to differentiate diseased tissue from normal surrounding tissue. Imaging involves the relationship between the three spatial dimensions of the region of interest and a fourth dimension, time, which relates to both the pharmacokinetics of the diagnostic agent and the period necessary to acquire the image. The physical properties that can be used to create an image signal include, e.g. emission or absorption of radiation, nuclear magnetic moments and relaxation, and transmission or reflection of ultrasound. Usually, the imaging of different organs and tissues for early detection and localization of numerous pathologies cannot be successfully achieved without using appropriate contrast agents.

Imaging contrast agents relate to substances, which are able to absorb certain types of signal much stronger than surrounding tissues. The contrast agents are specific for each imaging technique, and as a result of their accumulation in certain diseased sites of interest, those sites may be visualized when an appropriate imaging technique is applied. The tissue concentration that must be achieved for successful imaging varies between diagnostic techniques.

To facilitate the accumulation of contrast agents in the diseased zone, various microparticulates have been suggested as carriers for contrast agents. Among those microcarriers, liposomes, draw special attention because of their easily controlled properties. For almost two decades liposomes have been recognized as promising carriers for drugs and diagnostic agents for the following reasons: (1) Liposomes are completely biocompatible; (2) they can entrap practically any drug or diagnostic agent into either the internal water compartment or into the membrane itself depending on the physico-chemical properties of the compound; (3) liposome-incorporated compounds are protected from the inactivating effect in the body, yet at the same time do not cause undesirable side-reactions; (4) liposomes also provide a unique opportunity to deliver pharmaceuticals or diagostic agents into cells or even inside individual cellular compartments. Pursuing different *in vivo* delivery purposes, the size, charge and surface properties of liposomes can easily be changed simply by incorporation of different lipids and/or by variation of the preparation methods.

One area of important potential application of the new contrast-loaded liposomes is tumour imaging. It is well established that PLA₂ is secreted by malignant cells and immunohistochemical staining of various cancers, including cancer of pancreas, breast and stomach has shown increased levels of PLA₂. An increased expression and secretion of PLA₂ is also found in several cancer cell lines stimulated by inter-leukines such as IL-6. In addition, elevated extracellular PLA₂ activity has also been described in inflammatory and infected tissues.

The main mechanism of liposome accumulation in tumors is via extravasation through leaky tumor capillaries into the interstitial space. The tumour accumulation can be significantly increased by using long-circulating polymer coated liposomes.

The lipid-based microcarriers may also be used for visualization of inflammation and infection sites. Similar to what is known with respect to cancer tissue, the use of microparticulate imaging agents for the visualization of infection and inflammation sites characterized by elevated PLA₂ activity is based on the ability of microparticulates to accumulate via extravasation through leaky capillaries.

One of the important features of the novel diagnostic system is that certain lipid derivatives will be cleaved by extracellular PLA₂ in a well-defined manner in specific extracellular locations of diseased mammalian tissue characterized by an elevated activity of PLA₂. It has been found that extracellular PLA₂ is capable of cleaving monoether/monoester lipid derivatives so as to target the diagnostic label in the relevant diseased tissue. Degradation of the lipid derivatives and the liposomes by PLA₂ leads to a site-specific release of the diagnostic contrast agents in the diseased tissue.

### Targeting of infected tissues with lipid-based carriers composed of the novel and unnatural lipids

One of the important features of the targeted delivery systyem is the rapid uptake of liposomes *in vivo* by cells of the mononuclear phagocytic system (MPS). The MPS comprises the macrophages, one of the most important components of the immune system involved in the clearance of foreign particles, including liposomes. The macrophages reside in various organs and tissues, e.g. in the spleen and liver (Kupffer cells) and as free and fixed macrophages in the bone marrow and lymph nodes.

The novel lipid-based system composed of prodrug lipids is able to deliver various lipid prodrugs and encapsulated drugs directly to the diseased liver or spleen harbouring parasites due to an accumulation of the liposomes and an increased level of the prodrug cleaving PLA₂ enzyme in the infected tissues. One particular advantage of the lipid based drug delivery system is furthermore that extracellular PLA₂ activity is significantly increased towards organized lipid substrates such as the prodrug liposomes as compared to monomeric lipid substrates.

The released ether-lipid analogues that are generated by the action of PLA₂ have been shown to result in perturbation of key regulatory enzymes in parasites such as Leishmania and Trypanosoma (Lux et al. Biochem. Parasitology 111 (2000) 1-14) and are therefore toxic to the parasites if administered in sufficient amount. As the liver, spleen and bone marrow are organs and tissues wherein high concentrations of macrophages can be found, it is possible to achieve a targeted delivery of the toxic drugs to parasites inhabiting these organs. Furthermore, parasitic infections, which are characterized by elevated levels of PLA₂, such as the malaria causing parasites, are also targets for treatment with the novel prodrug liposomes. The elevated levels of PLA₂ in malaria infections caused by the parasite Plasmodium Falciparum has been described by e.g. Vadas et al. (Infection and Immunity, 60 (1992) 3928-3931 and Am. J. Trop. Hyg. 49 (1993) 455-459).

Normal cells typically possess ether-cleavage enzymes, which enable them to avoid the toxic effect of ether-lipids. However, some normal cells such as red blood cells have like cancer cells no means of avoiding the disruptive effect of the ether-lipids. Accordingly, therapeutic use of ether-lipids requires an effective drug delivery system that protects the normal cells from the toxic effects and is able to bring the ether-lipids directly to the diseased tissue.

Thus, the novel targeted prodrug delivery systems can be used for the treatment of parasitic infections, which is characterized by an increased level of PLA₂ in the infected tissue. This is achieved by administering an efficient amount (up to about 1000 mg per kg) of the liposomes wherein the active drug substance is present in the form of a lipid prodrug being a substrate for extracellular PLA₂. In addition, the prodrug liposomes are candidates for targeted transport of encapsulated conventional anti-parasitic drug(s), where a potentiation of the effectiveness of the conventional drug(s) in combination with the PLA₂ generated lysolipids and/or fatty acid derivatives might be obtainable.

### Toxicity

Toxicity of the liposomes comprising the lipid derivatives can be assessed by determining the therapeutic window "TW", which is a numerical value derived from the relationship between the compound's induction of hemolysis and its ability to inhibit the growth of tumour cells. TW values are defined as HI₅/GI₅₀ (wherein "HI₅" equals the concentration of compound inducing the hemolysis of 5% of the red blood cells in a culture, and wherein "GI₅₀" equals the dose of compound inducing fifty percent growth inhibition in a population of cells exposed to the agent). The higher an agent's HI₅ value, the less hemolytic is the agent - higher HI₅ 's mean that greater concentrations of compound are required to be present in order for the compound to induce 5% hemolysis. Hence, the higher its HI₅, the more therapeutically beneficial is a compound, because more of it can be given before inducing the same amount of hemolysis as an agent with a lower HI₅. By contrast, lower GI₅₀ 's indicate better therapeutic agents - a lower GI₅₀ value indicates that a lesser concentration of an agent is required for 50% growth inhibition. Accordingly, the higher is its HI₅ value and the lower is its GI₅₀ value, the better are a compound's agent's therapeutic properties.

Generally, when a drug's TW is less than 1, it cannot be used effectively as a therapeutic agent. That is, the agent's HI₅ value is sufficiently low, and its GI₅₀ value sufficiently high, that it is generally not possible to administer enough of the agent to achieve a sufficient level of tumour growth inhibition without also attaining an unacceptable level of hemolysis. As the lipid derivative liposomes take advantage of the lower extracellular PLA₂ activity in the bloodstream compared to the activity in the diseased tissue, it is believed that the TW will be much higher that for normal monoether lysolipids. As the variance in activity is in orders of magnitude and as the liposomes will be "trapped" in tissue with a high extracellular PLA₂ activity, it is generally believed the TW of the liposomes of the invention will be greater than about 3, more preferably greater than about 5, and still more preferably greater than about 8.

The invention will be illustrated by the following non-limiting example.

### EXAMPLE

A new and unnatural type of lipid analogs with the phosphocholine and phosphoglycerol head groups linked to the C-2 position of the glycerol moiety have been synthesized and the thermodynamic lipid membrane behavior has been investigated using differential scanning calorimetry. From the heat capacity measurements, it was observed that the pre-transition usually characterizing the lipid membrane phase behavior was abolished most likely due to the central position of the head groups providing better packing properties in the low temperature ordered gel phase. Activity measurements of secretory phospholipase A2 (PLA2) on unilamellar liposomal membranes revealed that the unnatural phospholipids are excellent substrates for PLA2 catalyzed hydrolysis. This was manifested as a minimum in the PLA2 lag time in the main phase transition temperature regime and a high degree of lipid hydrolysis over a broad temperature range. The obtained results provide new information about the interplay between the molecular structure of phospholipids and the lipid membrane packing constrains that govern the pre-transition. In addition, the PLA2 activity measurements are useful for obtaining deeper insight into the molecular details of the catalytic site of PLA2. The combined results also suggest new approaches to rationally design liposomal drug carries that can undergo a triggered activation in diseased tissue by overexpressed PLA2.

Fully hydrated lipid membranes composed of saturated phospholipids can undergo several thermotropic phase transitions, which are considered to be of relevance for the functional behavior of biological membranes [1,2,3]. In addition, the structural biomaterial properties of phospholipid membranes undergoing phase transitions are of importance for the development of new lipid based drug delivery systems with improved therapeutic effects [3,4,5,6,7]. The most extensively studied lipid membrane phase transition is the gel-to-fluid chain melting transition, which takes the membrane from a low temperature gel phase with the acyl chains in a predominantly ordered configuration to a fluid phase with the acyl chains in a highly disordered conformation [1,5]. The ripple phase, (*P*_{β}·), which is characterized by corrugations in the lipid membrane plane with a periodicity ranging from 10-30 nm, exists in a temperature range between the pre-transition and the main transition [8,9]. The ripple phase is one of the more intriguing ordered lamellar phases and its structural behavior has been extensively studied by a large number of experimental techniques including freeze fracture electron microscopy, differential scanning calorimetry, and atomic force microscopy [10,11,12,13,14,15]. The ripples have an asymmetric sawtooth profile with the acyl chains tilted with respect to the lipid membrane normal [10]. The molecular origin of ripple formation has traditionally been associated with the lipid head group region and for this reason phospholipids are normally divided into ripple forming lipids and non-ripple-forming lipids. One of the most extensively studied ripple forming lipid families is the phosphatidylcholine lipids [9,10,14]. Several attempts have been made to explain the reason for ripple phase formation in a lipid membrane. Examples of some of the molecular mechanisms that have been proposed to play an important role for the formation of a corrugated lipid membrane structure include an electrostatic coupling between the water molecules and the polar lipid head groups [16] and a coupling between membrane curvature and molecular tilt [17]. Other models assume that ripples form in order to relieve packing frustrations that arise whenever the relationship between the cross-sectional area of the bulky head group and the cross-sectional area of the long apolar tail exceeds a certain threshold value [18,19].

To demonstrate the present invention, we have synthesized a new class of unnatural phospholipid analogs where the phosphocholine (PC) and phosphoglycerol (PG) head groups are linked to the C-2 position and with an alkyl chain ether-linked to the C-1 position of the glycerol backbone of the phospholipids. Using differential scanning calorimetry, we have investigated the thermodynamic phase behavior and shown that the pre-transition that usually characterizes the thermodynamic lipid membrane phase behavior of natural PC and PG phospholipids is abolished for the unnatural phospholipid analogs. From the PLA2 activity measurements we have furthermore learned that these unnatural phospholipids behave as excellent substrates for PLA2 catalyzed hydrolysis. These findings are in agreement with earlier thermodynamic studies and PLA2 activity measurements carried out on 1,3-diacyl-*sn*-2-phosphocholines [20,21,22,23,24,25]. Our PLA2 activity measurements on the novel 1-O-DPPC' and 1-O-DPPG' liposomes furthermore revealed a minimum in the lag time in the gel-to-fluid transition region and a high degree of hydrolysis over a broad temperature range. Incorporation of small amounts of negatively charged PG phospholipids or DSPE-PEG₂₀₀₀ polymer lipids resulted in a reduction of the PLA2 lag time in accordance with earlier results on polymer grafted DPPC lipid membranes containing negatively charged polymer lipids [26]. In addition, human secretory PLA2 showed a high activity towards liposomes formed by the 1-O-DPPG' lipids.

Our combined approach involving chemical synthesis of new phospholipid analogs followed by thermodynamic bulk investigations of the lipid membrane phase behavior suggests new strategies to learn about molecular details of phospholipids that are of importance for ripple formation in a lipid membrane. Furthermore, the results provide information about the non-selective catalytic behavior of PLA2 that can be used, e.g. in combination with future molecular dynamic simulations, to map out conformational details of the catalytic active site of PLA2. In addition, the unnatural lipids offer new ways to rationally design soft lipid-based composite biomaterials such as liposomal drug carrier systems that can be degraded specifically by overexpressed PLA2 in diseased tissue [26,27,28,29,30].

The unnatural PC and PG phospholipids (1-O-DPPC', 1-O-DPPG') with the phosphocholine and phosphoglycerol head groups linked to the C-2 position of the glycerol moiety were synthesized utilizing (*S*)-O-benzyl glycidol as a versatile starting material [27,28] as shown in Fig. 2. Opening of the epoxide **1** under basic conditions, using THF/DMF (1:1) as a solvent system that minimizes dimerization gave **2** in 71% yield after purification by column chromatography. The phosphorylation was performed using phosphorous oxychloride in CH₂Cl₂ [27], which gave **3** in 65% yield. Debenzylation under H₂ atmosphere with Pd/C as catalyst followed by a simple acylation using palmitoyl chloride gave the target 1-O-DPPC' lipid. The synthesis of 1-O-DPPG' was carried out from **2** using (i-Pr)₂NPClOMe [31] as the phosphorylation reagent. The phosphorylation using TMP as base in the lipid coupling followed by (*R*)-isopropylidene glycerol with 5-phenyl-1H-tetrazole as a weak proton donor gave the protected phospholipid **4** in 67% yield after oxidation. Debenzylation followed by acylation using DCC gave **5** in 92% yield. Deprotection of lipid **5** was carried out with Me₃N to remove the methyl protection group, followed by stirring in CH₂Cl₂/MeOH/0.5M HCl (65:25:4) resulting in removal of the isopropylidene group. Finally, the proton on the phosphate was exchanged with sodium using NaHCO₃, which gave the desired 1-O-DPPG' in 70% yield after purification by column chromatography. AEL-43 and AEL-44 were obtained by simple deprotection. Debenzylation of **3** under H₂ with Pd/C as catalyst gave AEL-43 in quantitative yield. Deprotection of **4** was carried out using Me₃N, CH₂Cl₂/MeOH/0.5M HCl (65:25:4) as described above followed by debenzylation using H₂-Pd/C which gave AEL-44 in 71 % yield.

By replacing the ester linkage in the C-1 position with an ether bond, these unnatural phospholipids can furthermore be used as novel prodrug anticancer lipids to make liposomal microcarriers that can be degraded to active anticancer lysoetherlipids by PLA2 [27]. The lipid prodrug analogs (1-O-DPPC and 1-O-DPPG) were synthesized as described earlier [27,28]. 1,2-dipalmitoyl-*sn*-glycero-3-phosphocholine (DPPC), 1,2-dipalmitoyl-*sn*-glycero-3-phosphoglycerol (DPPG), and 1,2-distearoyl-*sn-*glycero-3-phosphoethanolamine poly(ethylene glycol)₂₀₀₀ (DSPE-PEG₂₀₀₀) were purchased from Avanti Polar Lipids, AL, USA. Multi- and unilamellar liposomes were hydrated in a HEPES buffer solution (10 mM HEPES, 110 mM KCI, 1 mM NaN₃, 30 µM CaCl₂, 10 µM NaEDTA, pH 7.5) and prepared as described in previous work [26,27]. Differential scanning calorimetry was carried out in the upscan mode at a scanrate of 20°C/h using multilamellar liposomes [26,27]. The PLA2 lag time measurements on 1-O-DPPC' unilamellar liposomes incorporated with small amounts of 1-O-DPPG' or DSPE-PEG₂₀₀₀ lipids were performed in the gel-to-fluid main phase transition range and the degree of lipid hydrolysis was analyzed using HPLC techniques [26,27,28].

The heat capacity (*C_{P}*) curves obtained using differential scanning calorimetry are shown in Fig. 3 for one-component 1-O-DPPC' and 1-O-DPPG' multilamellar liposomes. The C*_{P}*-curves show a main peak at 40.1°C and 40.6°C reflecting the chain disordering gel-to-fluid main phase transition of the 1-O-DPPG' and 1-O-DPPG' lipid membranes, respectively. As clearly demonstrated by the *C_{P}*-curves, the pretransition that usually characterizes the lipid membrane phase behavior of natural DPPC and DPPG lipids (Figs. 3A and 3B) is abolished for the unnatural 1-O-DPPC' and 1-O-DPPG' lipids. This leads to the formation of low temperature ordered and non-corrugated gel phase (*L*_{β}) that prevails until the unset of the main transition giving rise to a concomitant increase in the integrated melting enthalpy of the main transition (Table 1). Since the 1-O-DPPC' and 1-O-DPPG' lipids undergo a transition from an ordered configuration in the low temperature non-corrugated gel phase to a disordered configuration in the high temperature fluid phase without passing through the ripple phase, the integrated melting enthalpy is expected to be higher than for the natural DPPC and DPPG ripple phase forming lipids, which undergo a transition to the fluid phase from a ripple phase with a higher energy than the non-corrugated gel phase. In order to exclude the possibility that the abolished ripple transition is due to having an ether bond in the C-1 position, we have in Fig. 3 also shown *C_{P}*-curves obtained on 1-O-DPPC and 1-O-DPPG multilamellar liposomes. Clearly, these *C_{P}*-curves show the same characteristic phase behavior as the natural DPPC and DPPG lipids with a pre-transition a few degrees below the main phase transition peak underlining that it is the position of the phosphate head group in the C-3 position on the natural phospholipids that is crucial for ripple formation. Our results therefore suggest that ripples form in order to relieve packing frustrations that arise due to the flanked position of the bulky hydrophilic head group relative to the long apolar acyl chains on the natural DPPC and DPPG lipids, whereas an imbalance between the cross sectional area of the head and tail regions [18,19] and an electrostatic interaction between the water molecules and the polar lipid head groups [16] seem to be less crucial for ripple formation in line with results reported earlier on 1,3-diacyl-phosphocholine lipids [20,21].

Typical PLA2 (*A. piscivorus piscivorus*) hydrolysis time course profiles obtained at 40°C are shown in Fig. 4 for 1-O-DPPC' and 1-O-DPPG' unilamellar liposomes. The lag time, τ, shown in Fig. 4A is derived on basis of the time elapsed after addition of PLA2 to the lipid suspension until the unset of rapid lipid hydrolysis. The rapid increase in PLA2 activity is reflected by a sharp rise in the intrinsic PLA2 tryptophan fluorescence intensity [32,33] followed by a concomitant decrease in the 90° static light scattering caused by a change in the morphology of the lipid system when a large amount of the phospholipids are converted to non-bilayer forming lysolipids and free fatty acids [32,33]. It has earlier been argued that this lag time reflects the time required to slowly accumulate a threshold amount of lysolipid and fatty acid hydrolysis products in the lipid membrane needed to cause a sudden activation of PLA2 [32]. Both the fluorescence and light scattering data clearly indicate that the unnatural phospholipids are excellent substrates for PLA2 catalyzed hydrolysis. Apparently, these synthetic lipids are easily degradable by PLA2. This is especially seen for the negatively charged 1-O-DPPG' liposomes (Fig. 4B) where immediate hydrolysis is observed when PLA2 derived from human tear fluid was added to the suspension. A high activity of human secretory PLA2 is expected towards these negatively charged liposomes in accordance with earlier results showing that a threshold amount of negatively charged lipids is needed to activate the human PLA2 [42]. Figure 5 shows the lag time, τ, as a function of temperature in the gel-to-fluid transition range of unilamellar 1-O-DPPC' liposomes. The appearance of a miminum in the lag time at the gel-to-fluid phase transition is most likely caused by the formation of a heterogeneous membrane structure composed of fluctuating gel and fluid domains as earlier described for phosphocholine lipid membranes [26,27,33,34]. The results in Fig. 5 furthermore show that the PLA2 activity increases with increasing amounts of negatively charged 1-O-DPPG' lipids incorporated into the liposomes.

Since one of our aims was to develop new liposomal carriers that can be used for PLA2 triggered drug delivery [27,28] we have also tested the influence of DSPE-PEG₂₀₀₀ polymer lipids on PLA2 degradation. For drug delivery purposes it is necessary to incorporate a small amount of DSPE-PEG₂₀₀₀ lipids in order to obtain a sufficient blood circulation time [3]. When 5 mol% DPPE-PEG₂₀₀₀ lipopolymers are incorporated into the 1-O-DPPC' liposomes a very short lag time is observed as shown in Fig. 5 in accordance with similar studies using natural DPPC liposomes incorporated with DSPE-PEG₂₀₀₀ lipids [26]. Figure 6A shows the degree of PLA2 lipid hydrolysis 1000 s after the unset of the burst for the unnatural 1-O-DPPC' phospholipids containing 0 mol%, 2 mol% and 10 mol% 1-O-DPPG' lipids, and 5 mol% DPPE-PEG₂₀₀₀ polymer lipids. A remarkably high degree of lipid hydrolysis is observed over a broad temperature range clearly demonstrating that PLA2 is able to hydrolyze the unnatural 1-O-DPPC' and 1-O-DPPC' phospholipids. For the negatively charged 1-O-DPPC' lipids, which are superior substrates for the human secretory PLA2, the degree of lipid hydrolysis is furthermore determined as a function of time after addition of PLA2 to the lipid suspension (Fig. 6B). Although PLA2 is classified as an enzyme that is able to specifically catalyze the hydrolysis of the ester linkage in the C-2 position of natural phospholipids, our results demonstrate that the catalytic activity is sustained when the phosphate head group is linked to the C-2 position and the hydrolysable acyl chain is linked to the C-3 position in accordance with earlier work carried out on 1,3-diacyl-phosphocholine lipids [22,23,24]. For the 1,3-diacyl-phosphocholine lipids it has earlier been suggested that both acyl chains assume a bent conformation in the lipid membrane similar to the C-2 acyl chain in natural 1,2-diacyl-phosphocholine lipid substrates, and that it is this bent that is important for PLA2 hydrolysis [20]. Chupin and coworkers [43] have exploited PLA2 in the synthesis of platelet-activating factor analogs with the phosphate headgroup attached to the C-2 position.

The ability of PLEA2 to hydrolyze the unnatural phospholipids can advantageously in future studies in combination with, e.g. molecular dynamic simulations be used to learn more about the molecular details of the active site of PLA2 [35,36]. The non-selective behavior of PLA2 towards the natural/unnatural phospholipids furthermore raises an interesting question related to why natural lipids are designed as ripple forming lipids. Although our results are obtained on model lipid membranes, it is tempting to speculate that the ability of phospholipids to form ripple structures might be of importance for the functional and structural behavior of the lipid membrane part of cell membranes with locally ordered and differentiated lipid regions [37]. Interestingly, it has recently been demonstrated that PLA2 displays an increased activity towards the undulated ripple phase compared to the rather flat and non-corrugated gel phase [38].

In a broader perspective, the results presented above may have implications for a deeper understanding of the regulation of enzymatic activity via a modification of the structural phase behavior and physical properties of the lipid membrane part of biological membranes. Furthermore, a fundamental understanding of the influence of phospholipid composition on the functional biomaterial properties of lipid membranes is of relevance for a rational modification and optimization of liposomal drug carrier systems that can undergo a triggered activation [39,40] at diseased sites with high levels of PLA2 [29,30]. The unnatural phospholipids, which we have designed as prodrug lipids of anticancer lysolipids [27,28] can in addition be used as suitable building blocks to create novel liposomal prodrug and drug delivery systems that can undergo a PLA2 mediated degradation and activation [29,30].

The anticancer ether lipids AEL-43 and 44 were tested for their cytotoxicity against HT-29 colon cancer cells. It is very difficult to compare absolute values between different cytotoxicity experiments and AEL-43 and 44 were therefore tested with AEL-1,2,5 and 6 (Fig. 7). These reference compounds showed a higher IC₅₀-value compared to the values we have found earlier, and this quite large difference is difficult to explain. However, it could be a result of a higher cell concentration when initiating the experiment, which we have found to influence the experiments. The experiment was performed twice with similar results. Very interestingly, it was found that AEL-43 and 44 have IC₅₀-values that were 2-3 times lower than AEL-1 and 2. This result is very interesting as ET-18-OCH₃ and HePC normally have IC₅₀-values that are approximately 3 times lower [41].

Abbreviations: DPPC, 1,2-dipalmitoyl-sn-glycero-3-phosphocholine, 1,2-dihexadecanoyl-sn-glycero-3-phosphocholine; DPPG, 1,2-dipalmitoyl-sn-glycero-3-phosphoglycerol, 1,2-dihexadecanoyl-sn-glycero-3-phosphoglycerol; 1-O-DPPC', (S)-1-O-hexadecyl-3-hexadecanoyl-glycero-2-phosphocholine; 1-O-DPPG', (S)-1-O-hexadecyl-3-hexadecanoyl-glycero-2-phosphoglycerol; 1-O-DPPC, 1-O-hexadecyl-2-hexadecanoyl-sn-glycero-3-phosphocholine; 1-O-DPPG, 1-O-hexadecyl-2-hexadecanoyl-sn-glycero-3-phosphoglycerol; PLA2, phospholipase A2; DSPE-PEG₂₀₀₀, 1,2-distearoyl-sn-glycero-3-phosphoethanolamine poly(ethylene glycol)₂₀₀₀; AEL-1, 1-*O*-Hexadecyl-2-lyso-*sn*-glycero-3-phosphocholine; AEL-2, 1-*O*-Hexadecyl-2-lyso-*sn*-glycero-3-phospho-(*S*)-glycerol; AEL-5, 1-*O*-Octadecyl-2-lyso-*sn*-glycero-3-phosphocholine; AEL-6, 1-*O*-Octadecyl-2-lyso-*sn*-glycero-3-phospho-(*S*)-glycerol; AEL-43, (*R*)-1-*O*-Hexadecyl-3-lyso-glycero-2-phosphocholine; AEL-44, (*R*)-1-*O*-Hexadecyl-3-lyso-glycero-2-phospho-(*S*)-glycerol

### References

1. R. Lipowsky and E. Sackmann (Eds.), Handbook of Biological Physics. Structure and Dynamics of Membranes, Elsevier, Amsterdam, 1995, Vol. 1A&B, pp. 1-1020.
2. O. G. Mouritsen, K. Jorgensen, Small-scale lipid-membrane structure: simulation vs experiment, Curr. Opin. Struct. Biol. 7 (1997) 518-527.
3. D. D. Lasic, Liposomes: From Physics to Applications, Elsevier, Amsterdam, 1993.
4. D. Needham, M. W. Dewhirst, The development and testing of a new temperature-sensitive drug delivery system for the treatment of solid tumors, Adv. Drug Del. Rev. 53 (2001) 285-305.
5. O. G. Mouritsen, K. Jorgensen, A new look at lipid-membrane structure in relation to drug research, Pharm. Res. 15 (1998) 1507-1519.
6. D. D. Lasic, D. Needham, The "Stealth" liposome: a prototypical biomaterial, Chem. Rev. 95 (1995) 2601-2627.
7. R. Bittman, The 2003 ASBMB-Avanti award in lipids address: applications of novel synthetic lipids to biological problems, Chem. Phys. Lipids 129 (2004) 111-131.
8. E. J. Luna, H. M. McConnell, The intermediate monoclinic phase of phosphocholines, Biochim. Biophys. Acta 466 (1977) 381-392.
9. J. F. Nagle, S. Tristram-Nagle, Structure of lipid bilayers, Biochim. Biophys. Acta 1469 (2000) 159-195.
10.H. W. Meyer, Pretransition-ripples in bilayers of dipalmitoylphosphatidylcholine: Undulation or periodic segments? A freeze-fracture study, Biochim. Biophys. Acta 1302 (1996) 138-144.
11.D. Rüppel, E. Sackmann, On defects in different phases of two-dimensional lipid bilayers, J. Phys. 44 (1983) 1025-1034.
12.B. G. Tenchov, H. Yao, I. Hatta, Time-resolved X-ray-diffraction and calorimetric studies at low scan rates. Fully hydrated dipalmitoylphosphatidylcholine (DPPC) and DPPC/water ethanol phases, Biophys. J. 56 (1989) 757-768.
13.C. Trandum, P. Westh, K. Jørgensen, Slow relaxation of the sub-main transition in multilamellar phosphatidylcholine vesicles, Biochim. Biophys. Acta 1421 (1999) 207-212.
14.T. Kaasgaard, C. Leidy, J. H. Crowe, O. G. Mouritsen, K. Jørgensen, Temperature-controlled structure and kinetics of ripple phases in one- and two-component supported lipid bilayers, Biophys. J. 85 (2003) 350-360.
15.C. Leidy, T. Kaasgaard, J. H. Crowe, O. G. Mouritsen, K. Jørgensen, Ripples and the formation of anisotropic lipid domains: imaging two-component double bilayers by atomic force microscopy, Biophys. J. 83 (2002) 2625-2633.
16.S. Doniach, A thermodynamic model for the monoclinic (ripple) phase of hydrated phospholipids bilayers, J. Chem. Phys. 70 (1979) 4587-4596.
17.T. C. Lubensky, F. C. Mackintosh, Theory of ripple phases of lipid bilayers, Phys. Rev. Lett. 71 (1993) 1565-1568.
18.J. M. Carlson, J. P. Sethna, Theory of the ripple phase in hydrated phospholipid bilayers, Phys. Rev. A 36 (1987) 3359-3374.
19.S. Kirchner, G. Cevc, On the origin of thermal L(beta')-P(beta') pretransition in the lamellar phospholipid-membranes, Eur. Phys. Lett. 28 (1994) 31-36.
20.J. Seelig, R. Dijkman, G. H. De Haas, Thermodynamic and conformational studies on sn-2-phosphocholines in monolayers and bilayers, Biochemistry 19 (1980) 2215-2219
21.J. Stümpel, H. Eibl, A. Nicksch, X-ray analysis and calorimetry on phosphatidylcholine model membranes. The influence of length and position of acyl chains upon structure and phase behavior, Biochim. Biophys. Acta 727 (1983) 246-254.
22. G. H. de Haas, L. L. M. van Deenen, The stereospecific action of phospholipase A on β-lecithins, Biochim. Biophys. Acta 70 (1963) 469-471.
23. G. H. de Haas, L. L. M. van Deenen, The site of action ofphospholipase A on β-lecithins, Biochim. Biophys. Acta 84 (1964) 469-471.
24. A. J. Slotboom, R. Verger, H. M. Verheij, P. H. Baartmans, L. L. M. van Deenen, G. H. de Haas, Application of enantiomeric 2-sn-phosphatidylcholines in interfacial enzyme kinetic of lypolysis, Chem. Phys. Lipids 17 (1976) 128-147.
25. R. A. Dluhy, B. Z. Chowdhry, D. G. Cameron, Infrared characterization of conformational differences in lamellar phases of 1,3-dipalmitoyl-sn-glycerol-2-phosphocholine, Biochim. Biophys. Acta 821 (1985) 437-444.
26. J. Davidsen, K. Jørgensen, T. Andresen, O. G. Mouritsen, Secreted phospholipase A2 as a new principle for localised liposomal drug release and absorption in diseased tissue, Biochim. Biophys. Acta 1609 (2003) 95-101.
27. T. L. Andresen, J. Davidsen, M. Begtrup, O. G. Mouritsen, K. Jørgensen, Enzymatic release of anti-tumor ether lipids by specific phospholipase A2 action of novel liposome-forming prodrugs, J. Med. Chem. 47 (2004) 1694-1703.
28. S. S. Jensen, T. L. Andresen, J. Davidsen, P. Høyrup, S. D. Shnyder, M. C. Bibby, J. H. Gill, K. Jørgensen, Secretory phospholipase A2 as a tumor-specific trigger for targeted delivery of a novel class of liposomal prodrug anticancer etherlipids, Mol. Cancer Ther. 3 (2004) 1-8.
29. J. P. Laye, J. H. Gill, Phospholipase A2 expression in tumours: a target for therapeutic intervention, Drug Discov. Today 8 (2003) 710-716.
30. T. Abe, K. Sakamoto, H. Kamohara, Y. Hirano, N. Kuwahara, M. Ogawa, Group II phospholipase A2 is increased in peritoneal and pleural effusions in patients with various types of cancer, Int. J. Cancer 74 (1997) 245-250.
31. P. Brown, C. M. Richardson, L. M. Mensah, P. J. O'Hanlon, N. F. Osborne, A. J. Pope, G. Walker, Molecular recognition of tyrosinyl adenylate analogues by prokaryotic tyrosyl tRNA synthetases, Bioorg. Med. Chem. 7 (1999) 2473-85.
32. W. R. Burack, Q. Yuan, R. L. Biltonen, Role of lateral phase separation in the modulation of phospholipase A2 activity, Biochemistry 32 (1993) 583-589.
33. T. Hønger, K. Jørgensen, R. L. Biltonen, O. G. Mouritsen, Systematic relationship between phospholipase A2 activity and dynamic lipid bilayer microhetero-geneity, Biochemistry 35 (1996) 9003-9006.
34. J. A. F. Op den Kamp, J. de Gier, L. L. M. van Deenen, Hydrolysis of phosphatidylcholine liposomes by pancreatic phospholipase A2 at the transition temperature, Biochim. Biophys. Acta 345 (1974) 253-256.
35. B. Waszkowycz, J. H. Hillier, N. Gensmantel, D. W. Payling, A quantum mechanical/molecular mechanical model of inhibition of the enzyme phospholipase A2, J. Chem. Soc. Perkin. Trans. 2 (1991) 1819-1832.
36. E. D. Mihelich, R. W. Schevitz, Structure-based design of a new class of anti-inflammatory drugs: secretory phospholipase A2 inhibitors, SPI, Biochim. Biophys. Acta 1441 (1999) 223-228.
37. D. A. Brown, E. London, Structure of detergent-resistant membrane domains: does phase separation occur in biological membranes? Biochem. Biophys. Res. Commun. 240 (1997) 1-7.
38. C. Leidy, O. G. Mouritsen, K. Jørgensen, G. H. Peters, Evolution of a rippled membrane during phospholipase A2 hydrolysis studied by time-resolved AFM, Biophys. J. 87 (2004) 408-418.
39. Y. Barenholz, Liposome application: problems and prospects, Cur. Opin. Colloid. Interface Sci. 6 (2001) 66-77.
40. T. M. Allen, P. R. Cullis, Drug delivery systems: entering the mainstream, Science 303 (2004) 1818-1822.
41. T. Wieder, W. Reutter, C. E. Orfanos, C. C. Geilen, Mechanisms of action of phospholipid analogs as anticancer compounds, Prog. Lip. Res. 38 (1999) 249-259.
42. A. G. Buckland, E. L. Heeley, D. C. Wilton, Bacterial cell membrane hydrolysis by secreted phospholipases A(2): a major physiological role of human group IIA sPLA(2) involving both bacterial cell wall penetration and interfacial catalysis, Biochem. Biophys. Acta 1484 (2000) 195-206.
43. V. V. Chupin, O. V. Ostapenko, V. N. Klykov, M. V. Anikin, G. A. Serebren-nikova, On the synthesis of platelet-activating factor via acylation of 1-alkyl-sn-glycero-3-phosphocholine. Formation of structural isomer of PAF in the presence of bases, Chem. Phys. Lipids 81 (1996) 35-43.

## Claims

1. A lipid based drug delivery system for administration of a drug substance, wherein the (a) drug substance is incorporated in the system said system including lipid derivatives which an aliphatic group of a length of at least 7 carbon atoms and an organic radical having at least 7 carbon atoms, and (b) a hydrophilic moiety, where the lipid derivative furthermore is a substrate for extracellular phospholipase A2 to the extent that the organic radical can be hydrolytically cleaved off, said system having included therein lipopolymers or glycolipids so as to present hydrophilic chains on the surface of the system, and wherein the lipid derivative is a lipid derivative of the following formula: 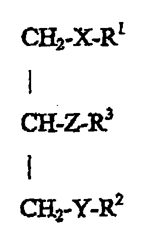 wherein
X and Z independently are selected from O, CH₂, NH, NMe, S, S(O), OS(O), S(O)₂, OS(O)₂, OP(O)₂, OP(O)₂O, OAs(O)₂ and OAs(O)₂O;
Y is selected from OC(O), OC(O)O, OC(O)N, OC(S), SC(O), SC(S), CH₂C(O)O, NC(O)O, Y then being connected to R² via either the oxygen, sulphur, nitrogen or carbonyl carbon atom;
R¹ is an aliphatic group of the formula Y¹Y²;
R² is an organic radical having at least 2 carbon atoms;
where Y¹ is -(CH₂)ₙ₁-(CH=CH)ₙ₂-(CH₂)ₙ₃-(CH=CH)ₙ₄-(CH₂)ₙ₅-(CH₂)ₙ₇-(CH=CH)ₙ₈-(CH₂)ₙ₉, and the sum of n1+2n2+n3+2n4+n5+2n6+n7+2n8+n9 is an integer of from 2 to 29; n1 is zero or an integer from 1 to 29, n3 is zero or an integer from 1 to 20, n5 is zero or an integer from 1 to 17, n7 is zero or an integer from 1 to 14, and n9 is zero or an integer from I to 11; and each of n2, n4, n6 and n8 is independently zero or 1; and Y² is CH₃, CO₂H, SH, S(O)₂OH, P(O)₂OH, OP(O)₂OH, OH, NH₂; where each carbon of Y¹-Y² independently may be substituted with halogens and aliphatic substituents,
R³ is selected from phosphatidic acid (PO₂-OH), derivatives of phosphatidic acid and bioisosters to phosphate acid and derivatives thereof.

2. The lipid based drug delivery system according to claim 1, wherein the aliphatic group remains substantially unaffected, so as to result in an organic acid fragment or an organic alcohol fragment and a lysolipid fragment.

3. The lipid based drug delivery system according to claim 1, wherein the lipopolymers or glycolipids are represented by a fraction of the lipid derivative.

4. The lipid based drug delivery system according to any of the claims 1-3, wherein the polymer of the lipopolymer is selected from polyethylene glycol, poly(lactic acid), poly(glycolic acid), poly(lactic acid)-poly(glycolic acid) copolymers, polyvinyl alcohol, polyvinylpyrrolidone, polymethoxazoline, polyethyloxazoline, polyhydroxypropyl methacrylamide, polymethacrylamide, polydimethylacrylamide, and derivatised celluloses.

5. The lipid based drug delivery system according to any of the claims 1-4, wherein the organic radical which can be hydrolytically cleaved off, is an auxiliary drug substance or an efficiency modifier for the second drug substance.

6. The lipid based drug delivery system according to claim 1-5, wherein R² is an aliphatic group of a length of at least 7 carbon atoms.

7. The lipid based drug delivery system according to claim 6, wherein R² is a group of the formula Y¹Y².

8. The lipid based drug delivery system according to any of the claims 1-7, wherein at least a fraction of the lipid is of the formula defined in claims 6, wherein R³ is a derivative of phosphatidic acid to which a polymer selected from polyethylene glycol, poly(lactic acid), poly(glycolic acid), poly(lactic acid)-poly(glycolic acid) copolymers, polyvinyl alcohol, polyvinylpyrrolidone, polymethoxazoline, polyethyloxazoline, polyhydroxypropyl methacrylamide, polymethacrylamide, polydimethylacrylamide, and derivatised celluloses, is covalently attached.

9. The lipid based drug delivery system according to any of the claims 1-8, wherein the lipid derivative constitutes 5-100 mol% of the total dehydrated system.

10. The lipid based drug delivery system according to any of the claims 1-9, wherein the lipopolymer constitutes 2-50 mol% of the total dehydrated system.

11. The lipid based drug delivery system according to any of the claims 1-10, wherein the system is in the form of liposomes.

12. The lipid based drug delivery system according to any of the claims 1-11, wherein the second drug substance is a therapeutically and/or prophylactically active substance selected from (i) antitumor agents, (ii) antibiotics and antifungals, and (iii) antiinflammatory agents.

13. A pharmaceutical composition comprising the lipid based drug delivery system according to any of the claims 1-12 and optionally a pharmaceutically acceptable carrier.

14. The lipid based drug delivery system according to any of the claims 1-12 for use as a medicament.

15. Use of a drug delivery system according to any of the claims 1-12 for the preparation of a medicament for the treatment of diseases or conditions associated with a localised increase in extracellular phospholipase A2 activity in mammalian tissue.

16. The use according to claim 15, wherein the diseases or conditions are selected from the group consisting of inflammatory conditions and cancer.

17. The use according to claim 16, wherein the type of cancer is selected from the group consisting of brain cancer, breast cancer, lung cancer, colon cancer, ovarian cancer, leukemia, lymphoma, sarcoma and carcinoma.

18. The use according to any of claims 16-17, wherein the increase in extracellular phospholipase A2 activity is a least 25% compared to the normal level of activity in the tissue in question.

19. The lipid based drug delivery system according to any of the claims 1-5, wherein the lipid derivative is a lipid derivative of the following formula: 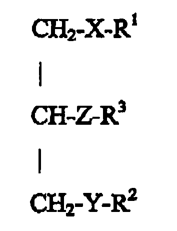 wherein
X = Y;
Y is selected from OC(O), OC(O)O, OC(O)N, OC(S), SC(O), SC(S), CH₂C(O)O, NC(O)O, Y then being connected to R² via either the oxygen, sulphur, nitrogen or carbonyl carbon atom;
Z is selected from O, CH₂, NH, NMe, S, S(O), OS(O), S(O)₂, OS(O)₂, OP(O)₂, OP(O)₂O, OAs(O)₂ and OAs(O)₂O;
R¹ is an aliphatic group of the formula Y¹Y²;
R² is an organic radical having at least 2 carbon atoms;
where Y¹ is -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈-(CH₂)ₙ₉, and the sum of n1+2n2+n3+2n4+n5+2n6+n7+2n8+n9 is an integer of from 2 to 29; n1 is zero or an integer of from 1 to 29, n3 is zero or an integer of from 1 to 20, n5 is zero or an integer of from 1 to 17, n7 is zero or an integer of from 1 to 14, and n9 is zero or an integer of from I to 11; and each of n2, n4, n6 and n8 is independently zero or 1; and Y² is CH₃, CO₂K SH, S(O)₂OH, P(O)₂OH, OP(O)₂OH, OH, NH₂; where each carbon of Y¹-Y² independently may be substituted with halogens and aliphatic substituents,
R³ is selected from phosphatidic acid (PO₂-OH), derivatives of phosphatidic acid and bioisosters to phosphatic acid and derivatives thereof.

20. The lipid based drug delivery system according to claim 19, wherein R² is an aliphatic group of a length of at least 7 carbon atoms.

21. The lipid based drug delivery system according to claim 6, wherein R² is a group of the formula Y¹Y².

22. The lipid based drug delivery system according to claims 1 and 21, wherein the system is in the form of liposomes.

23. The lipid based drug delivery system according to any of the claims 1-5 and 19-22 , wherein the drug substance is a therapeutically and/or prophylactically active substance selected from (i) antitumor agents, (ii) antibiotics and antifungals, and (iii) antiinflammatory agents.

24. A Pharmaceutical composition comprising the drug delivery system according to any of the claims 1-5, 19-23 and optionally a pharmaceutically acceptable carrier.

25. The lipid based drug delivery system according to any of the claims 1-5, 19-23 for use as a medicament

26. The use of a drug delivery system according to any of the claims 1-5, 19-23 for the preparation of a medicament for the treatment of diseases or conditions associated with a localised increase in extracellular phospholipase A2 activity in mammalian tissue.

27. The use according to claim 26, wherein the diseases or conditions are selected from the group consisting of inflammatory conditions and cancer.

28. The use according to claim 27, wherein the type of cancer is selected from the group consisting of brain cancer, breast cancer, lung cancer, colon cancer, ovarian cancer, leukemia, lymphoma, sarcoma and carcinoma.

29. The use according to any of claims 26-27, wherein the increase in extracellular phospholipase A2 activity is a least 25% compared to the normal level of activity in the tissue in question.

## Patentansprüche

1. Arzneimittclabgabesystem auf Lipidbasis zur Gabe eines Arzneistoffs, wobei der (a) Arzneistoff in das System integriert ist, wobei das System Lipid-Derivate mit einer aliphatischen Gruppe einer Länge von zumindest 7 Kohlenstoffatomen und einem organischen Radikal mit zumindest 7 Kohlenstoffatomen einschließt, und (b) ein hydrophiler Teil, wobei das Lipid-Derivat weiterhin insoweit ein Substrat für extrazelluläre Phospholipase A2 ist, als das organische Radikal hydrolytisch abgespalten werden kann, wobei das System Lipopolymere oder Glycolipide einschließt, damit hydrophile Ketten an der Oberfläche des Systems vorliegen, und wobei das Lipid-Derivat ein Lipid-Derivat der folgenden Formel ist: 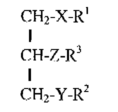 wobei
X und Z unabhängig voneinander ausgewählt sind aus O, CH₂, NH, NMe, S, S(O), OS(O), S(O)₂, OS(O)₂, OP(O)₂, OP(O)₂O, OAs(O)₂ und OAs(O)₂O;
Y ausgewählt ist aus OC(O), OC(O)O, OC(O)N, OC(S), SC(O), SC(S), CH₂C(O)O, NC(O)O, wobei Y dann entweder über das Sauerstoff-, Schwefel-, Stickstoff- oder Carbonyl-Kohlenstoffatom an R² gebunden ist;
R¹ eine aliphatische Gruppe der Formel Y¹Y² ist;
R² ein organisches Radikal mit zumindest 2 Kohlenstoffatomen ist;
wobei Y¹ -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈ -(CH₂)ₙ₉ ist, und die Summe aus n1+2n2+n3+2n4+n5+2n6+n7+2n8+n9 eine ganze Zahl von 2 bis 29 ist, n1 null oder eine ganze Zahl von 1 bis 29 ist, n3 null oder eine ganze Zahl von 1 bis 20 ist, n5 null oder eine ganze Zahl von 1 bis 17 ist, n7 null oder eine ganze Zahl von 1 bis 14 ist und n9 null oder eine ganze Zahl von 1 bis 11 ist und n2, n4, n6 und n8 jeweils unabhängig voneinander null oder 1 sind und Y² CH₃, CO₂H, SH, S(O)₂OH, P(O)₂OH, OP(O)₂OH, OH, NH₂ ist, wobei jeder Kohlenstoff von Y¹-Y² unabhängig mit Halogenen und aliphatischen Substituenten substituiert sein kann;
R³ ausgewählt ist aus Phosphatidsäure (PO₂-OH), Derivaten der Phosphatidsäure und Bioisosteren zur Phosphatidsäure und Derivaten davon.

2. Arzneimittelabgabesystem auf Lipidbasis nach Anspruch 1, wobei die aliphatische Gruppe im Wesentlichen unbeeinflusst bleibt, so dass sich ein organischer Säurerest oder ein organischer Alkoholrest und ein Lysolipidrest ergeben.

3. Arzneimittelabgabesystem auf Lipidbasis nach Anspruch 1, wobei die Lipopolymere oder Glycolipide durch einen Rest des Lipid-Derivats dargestellt werden.

4. Arzneimittelabgabesystem auf Lipidbasis nach einem der Ansprüche 1 bis 3, wobei das Polymer des Lipopolymers ausgewählt ist aus Polyethylenglycol, Poly(milchsäure), Poly(glycolsäure), Poly(milchsäure)-Poly(glycolsäure)-Copolymeren, Yolyvinylalkohol, Polyvinylpyrrolidon, Polymethoxazolin, Polyethyloxazolin, Polyhydroxypropylmethacrylamid, Polymethacrylamid, Polydimethylacrylamid und derivatisierten Cellulosen.

5. Arzneimittelabgabesystem auf Lipidbasis nach einem der Ansprüche 1 bis 4, wobei das organische Radikal, das hydrolytisch abgespalten werden kann, ein Hilfsarmeistoff oder ein effiziente Modifikator für den zweiten Arzneistoff ist.

6. Arzneimittelabgabesystem auf Lipidbasis nach Anspruch 1 bis 5, wobei R² eine aliphatische Gruppe einer Länge von zumindest 7 Kohlenstoffatomen ist.

7. Arzneimittelabgabesystem auf Lipidbasis nach Anspruch 6, wobei R² eine Gruppe der Formel Y¹Y² ist.

8. Arzneimittetabgabesystem auf Lipidbasis nach einem der Ansprüche 1 bis 7, wobei zumindest ein Teil des Lipids die in Anspruch 6 definierte Formel aufweist, wobei R³ ein Derivat der Phosphatidsäure ist, an das ein Polymer, ausgewählt aus Polyethylenglycol, Poly(milchsäure), Poly(glycolsäure), Poly(milchsäure)-Poly(glycolsäure)-Copolymeren, Polyvinylalkohol, Polyvinylpyrrolidon, Polymethoxazolin, Polyethyloxazolin, Polyhydroxypropylmethacrylamid, Polymethacrylamid, Polydimethylacrylamid und derivatisierten Cellulosen, kovalent gebunden ist.

9. Arzneimittelabgabesystem auf Lipidbasis nach einem der Ansprüche 1 bis 8, wobei das Lipid-Derivat 5 bis 100 Mol% des gesamten dehydratisierten Systems darstellt.

10. Arzneimittelabgabesystem auf Lipidbasis nach einem der Ansprüche 1 bis 9, wobei das Lipopolymer 2 bis 50 Mol% des gesamten dehydratisierten Systems darstellt.

11. Arzneimittelabgabesystem auf Lipidbasis nach einem der Ansprüche 1 bis 10, wobei das System in Form von Liposomen vorliegt.

12. Arzneimittelabgabesystem auf Lipidbasis nach einem der Ansprüche 1 bis 11, wobei der zweite Arzneistoff eine therapeutisch und/oder prophylaktisch wirksame Substanz ist, ausgewählt aus (i) Antitumormitteln, (ii) Antibiotika und Antimykotika und (iii) entzündungshemmenden Mitteln.

13. Pharmazeutische Zusammensetzung, umfassend das lipidbasierte Arzneimittelabgabesystem nach einem der Ansprüche 1 bis 12 und wahlweise einen pharmazeutisch akzeptablen Träger.

14. Arzneimittelabgabesystem auf Lipidbasis nach einem der Ansprüche 1 bis 12 zur Verwendung als Medikament.

15. Verwendung eines Arzneimittelabgabesystems nach einem der Ansprüche 1 bis 12 zur Herstellung eines Medikaments für die Behandlung von Erkrankungen oder Zuständen, die mit einer lokalen Zunahme der Aktivität extrazellulärer Phospholipase A2 in Säugergewebe verbunden sind.

16. Die Verwendung nach Anspruch 15, wobei die Erkrankungen oder Zustände ausgewählt sind aus der Gruppe bestehend aus entzündliche Zuständen und Krebs.

17. Die Verwendung nach Anspruch 16, wobei die Krebsart ausgewählt ist aus der Gruppe bestehend aus Hirnkrebs, Brustkrebs, Lungenkrebs, Dickdarmkrebs, Eierstockkrebs, Leukämie, Lymphom, Sarkom und Karzinom.

18. Die Verwendung nach einem der Ansprüche 16 bis 17, wobei die Zunahme der Aktivität extrazellulärer Phospliolipase A2 zumindest 25 % im Vergleich zum normalen Aktivitätsniveau in dem betreffenden Gewebe beträgt.

19. Arzneimittelabgabesystem auf Lipidbasis nach einem der Ansprüche 1 bis 5, wobei das Lipid-Derivat ein Lipid-Derivat der folgenden Formel ist: 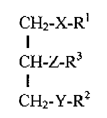 wobei
X = Y,
Y ausgewählt ist aus OC(O), OC(O)O, OC(O)N, OC(S), SC(O), SC(S), CH₂C(O)O, NC(O)O, wobei Y dann entweder über das Sauerstoff-, Schwefel-, Stickstoff- oder Carbonyl-Kohlenstoffatom an R² gebunden ist;
Z ausgewählt ist aus O, CH₂, NH, NMe, S, S(O), OS(O), S(O)₂, OS(O)₂, OP(O)₂, OP(O)₂O, OAs(O)₂ und OAs(O)₂O;
R¹ eine aliphatische Gruppe der Formel Y¹Y² ist;
R² ein organisches Radikal mit zumindest 2 Kohlenstoffatomen ist;
wobei Y¹ -(CH₂)ₙ₁-(CH-CH)ₙ₂-(CH₂)ₙ₃-(CH=CH)ₙ₄-(CH₂)ₙ₅-(CH-CH)ₙ₆-(CH₂)ₙ₇-(CH-CH)ₙ₈ -(CH₂)ₙ₉ ist, und die Summe aus n1+2n2+n3+2n4+n5+2n6+n7+2n8+n9 eine ganze Zahl von 2 bis 29 ist, n1 null oder eine ganze Zahl von 1 bis 29 ist, n3 null oder eine ganze Zahl von 1 bis 20 ist, n5 null oder eine ganze Zahl von 1 bis 17 ist, n7 null oder eine ganze Zahl von 1 bis 14 ist und n9 null oder eine ganze Zahl von 1 bis 11 ist und n2, n4, n6 und n8 jeweils unabhängig voneinander null oder 1 sind und Y² CH₃, CO₂H, SH, S(O)₂OH, P(O)₂OH, OP(O)₂OH, OH, NH₂ ist, wobei jeder Kohlenstoff von Y¹-Y² unabhängig mit Halogenen und aliphatischen Substituenten substituiert sein kann;
R³ ausgewählt ist aus Phosphatidsäure (PO₂-OH), Derivaten der Phosphatidsäure und Bioisosteren zur Phosphatidsäure und Derivaten davon.

20. Arzneimittelabgabesystem auf Lipidbasis nach Anspruch 19, wobei R² eine aliphatische Gruppe einer Länge von zumindest 7 Kohlenstoffatomen ist.

21. Arzneimittelabgabesystem auf Lipidbasis nach Anspruch 6, wobei R² eine Gruppe der Formel Y¹Y² ist.

22. Arzneimittelabgabesystem auf Lipidbasis nach Anspruch 1 und 21, wobei das System in Form von Liposomen vorliegt.

23. Arzneimittelabgabesystem auf Lipidbasis nach einem der Ansprüche 1 bis 5 und 19 bis 22, wobei der zweite Arzneistoff eine therapeutisch und/oder prophylaktisch wirksame Substanz ist, ausgewählt aus (i) Antitumormitteln, (ii) Antibiotika und Antimykntika und (iii) entzündungshemmenden Mitteln.

24. Pharmazeutische Zusammensetzung, umfassend das lipidbasierte Arzneimittelabgabesystem nach einem der Ansprüche 1 bis 5, 19 bis 23 und wahlweise einen pharmazeutisch akzeptablen Träger.

25. Arzneimittelabgabesystem auf Lipidbasis nach einem der Ansprüche 1 bis 5, 19 bis 23 zur Verwendung als Medikament.

26. Die Verwendung eines Arzneimittelabgabesystems nach einem der Ansprüche 1 bis 5, 19 bis 23 zur Herstellung eines Medikaments für die Behandlung von Erkrankungen oder Zuständen, die mit einer lokalen Zunahme der Aktivität extrazellulärer Phospholipase A2 in Säugergewebe verbunden sind.

27. Die Verwendung nach Anspruch 26, wobei die Erkrankungen oder Zustände ausgewählt sind aus der Gruppe bestehend aus entzündlichen Zuständen und Krebs.

28. Die Verwendung nach Anspruch 27, wobei die Krebsart ausgewählt ist aus der Gruppe bestehend aus Himkrebs, Brustkrebs, Lungenkrebs, Dickdarmkrebs, Eierstockkrebs, Leukämie, Lymphom, Sarkom und Karzinom.

29. Die Verwendung nach einem der Ansprüche 26 bis 27, wobei die Zunahme der Aktivität extrazellulärer Phospholipase A2 zumindest 25 % im Vergleich zum normalen Aktivitätsniveau in dem betreffenden Gewebe beträgt.

## Revendications

1. Système d'apport de médicament à base de lipide destiné à l'administration d'une substance médicamenteuse, où la (a) substance médicamenteuse est incorporée dans le système, ledit système incluant des dérivés lipidiques qui présentent un groupe aliphatique d'une longueur d'au moins 7 atomes de carbone et un radical organique ayant au moins 7 atomes de carbone, et (b) une fraction hydrophile, où le dérivé lipidique est en outre un substrat de la phospholipase A2 extracellulaire dans une mesure telle que le radical organique peut être clivé par hydrolyse, ledit système contenant des lipopolymères ou glycolipides inclus de façon à présenter des chaînes hydrophiles à la surface du système, et où le dérivé lipidique est un dérivé lipidique de formule suivante : 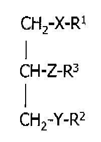 où
X et Z sont indépendamment sélectionnés parmi 0, CH₂, NH, NMe, S, S(O), OS(O), S(O)₂, OS(O)₂, OP(O)₂, OP(O)₂O, OAS(O)₂ et OAs(O)₂O ;
Y est sélectionné parmi OC(O), OC(O)O, OC(O)N, OC(S), SC(O), SC(S), CH₂C(O)O, NC(O)O, Y étant alors relié à R² soit par l'oxygène, le soufre, l'azote, soit par un atome de carbone d'un groupe carbonyle ;
R¹ est un groupe aliphatique de formule Y¹Y² ;
R² est un radical organique ayant au moins deux atomes de carbone ;
où Y¹ est -(CH₂)ₙ₁-(CH=CH)ₙ₂-(CH₂)ₙ₃-(CH=CH)ₙ₄-(CH₂)ₙ₅-(CH=CH)ₙ₆-(CH₂)ₙ₇-(CH=CH)ₙ₈-(CH₂)ₙ₉, et la somme de n1+2n2+n3+2n4+n5+2n6+n7+2n8+n9 est un entier de 2 à 29 ; n1 est zéro ou un entier de 1 à 29, n3 est zéro ou un entier de 1 à 20, n5 est zéro ou un entier de 1 à 17, n7 est zéro ou un entier de 1 à 14 et n9 est zéro ou un entier de 1 à 11 ; et chacun de n2, n4, n6 et n8 est indépendamment zéro ou 1 ; et Y² est CH₃, CO₂H, SH, S(O)₂OH, P(O)₂OH, OP(O)₂OH, OH, NH₂; où chaque carbone de Y¹-Y² peut être indépendamment substitué par des halogènes et des substituants aliphatiques,
R³ est sélectionné parmi l'acide phosphatidique (PO₂-OH), des dérivés de l'acide phosphatidique et des bioesters de l'ester phosphatidique et leurs dérivés.

2. Système d'apport de médicament à base de lipide selon la revendication 1, où le groupe aliphatique demeure sensiblement non affecté de façon à donner un fragment acide organique ou un fragment alcool organique et un fragment lysolipidique.

3. Système d'apport de médicament à base de lipide selon la revendication 1, où les lipopolymères ou glycolipides sont représentés par une fraction du dérivé lipidique.

4. Système d'apport de médicament à base de lipide selon l'une quelconque des revendications 1 à 3, où le polymère du lipopolymère est sélectionné parmi le polyéthylèneglycol, le poly(acide lactique), le poly(acide glycolique), des copolymères poly(acide lactique)-poly(acide glycolique), le poly(alcool vinylique), la polyvinyipyrrolidone, la polyméthoxazoline, la polyéthyloxazoline, le polyhydroxypropylméthacryl-amide, le polyméthacrylamide, le polydiméthylacrylamide et des celluloses dérivées.

5. Système d'apport de médicament à base de lipide selon l'une quelconque des revendications 1 à 4, où le radical organique qui peut être clivé par hydrolyse est une substance médicamenteuse auxiliaire ou un modificateur d'efficacité de la seconde substance médicamenteuse.

6. Système d'apport de médicament à base de lipide selon la revendication 1 à 5, où R² est un groupe aliphatique d'une longueur d'au moins 7 atomes de carbone.

7. Système d'apport de médicament à base de lipide selon la revendication 6, où R² est un groupe de formule Y¹Y².

8. Système d'apport de médicament à base de lipide selon l'une quelconque des revendications 1 à 7, où au moins une fraction du lipide est de formule définie à la revendication 6, où R³ est un dérivé de l'acide phosphatidique auquel est fixé par covalence un polymère sélectionné parmi le polyéthylèneglycol, le poly(acide lactique), le poly(acide glycolique), des copolymères poly(acide lactique)-poly(acide glycolique), le poly(alcool vinylique), la polyvinylpyrrolidone, la polyméthoxazoline, la polyéthyloxazoline, le polyhydroxypropylméthacryl-amide, le polyméthacrylamide, le polydiméthylacrylamide et des celluloses dérivées.

9. Système d'apport de médicament à base de lipide selon l'une quelconque des revendications 1 à 8, où le dérivé lipidique constitue 5 à 100 % en mole du système total déshydraté.

10. Système d'apport de médicament à base de lipide selon l'une quelconque des revendications 1 à 9, où le lipopolymère constitue 2 à 50 % en mole du système total déshydraté.

11. Système d'apport de médicament à base de lipide selon l'une quelconque des revendications 1 à 10, où le système est sous la forme de liposomes.

12. Système d'apport de médicament à base de lipide selon l'une quelconque des revendications 1 à 11, où la seconde substance médicamenteuse est une substance thérapeutiquement et/ou prophylactiquement active, sélectionnée parmi (i) des agents antitumoraux, (ii) des antibiotiques et des antifongiques, et (iii) des agents anti-inflammatoires.

13. Composition pharmaceutique comprenant le système d'apport de médicament à base de lipide selon l'une quelconque des revendications 1 à 12 et facultativement un véhicule pharmaceutiquement acceptable.

14. Système d'apport de médicament à base de lipide selon l'une quelconque des revendications 1 à 12 pour utilisation en tant que médicament.

15. Utilisation d'un système d'apport de médicament selon l'une quelconque des revendications 1 à 12 pour la préparation d'un médicament destiné au traitement de maladies ou de pathologies associées à une élévation localisée de l'activité de la phospholipase A2 extracellulaire dans le tissu mammalien.

16. Utilisation selon la revendication 15, où les maladies ou pathologies sont sélectionnées dans le groupe constitué par des pathologies inflammatoires et le cancer.

17. Utilisation selon la revendication 16, où le type de cancer est sélectionné dans le groupe constitué par le cancer du cerveau, le cancer du sein, le cancer du poumon, le cancer du côlon, le cancer ovarien, la leucémie, les lymphomes, les sarcomes et les carcinomes.

18. Utilisation selon l'une quelconque des revendications 16 à 17, où l'élévation de l'activité de la phospholipidase A2 extracellulaire est d'au moins 25 % par comparaison au taux normal d'activité dans le tissu en question.

19. Système d'apport de médicament à base de lipide selon l'une quelconque des revendications 1 à 5, où le dérivé lipidique est un dérivé lipidique de formule suivante : 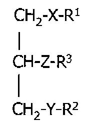 où
X = Y ;
Y est sélectionné parmi OC(O), OC(O)O, OC(O)N, OC(S), SC(O), SC(S), CH₂C(O)O, NC(O)O, Y étant alors relié à R² soit par l'oxygène, le soufre, l'azote, soit par un atome de carbone d'un groupe carbonyle ;
Z est sélectionné parmi 0, CH₂, NH, NMe, S, S(O), OS(O), S(O)₂, OS(O)₂, OP(O)₂, OP(O)₂O, OAS(O)₂ et OAs(O)₂O ;
R¹ est un groupe aliphatique de formule Y¹Y² ;
R² est un radical organique ayant au moins deux atomes de carbone ;
où Y¹ est -(CH₃)ₙ₁-(CH-CH)ₙ₂-(CH₂)ₙ₃-(CH-CH)ₙ₄-(CH₂)ₙ₅-(CH=CH)ₙ₆-(CH₂)ₙ₇-(CH=CH)ₙ₈-(CH₂)ₙ₉, et la somme de n1+2n2+n3+2n4+n5+2n6+n7+2n8+n9 est un entier de 2 à 29 ; n1 est zéro ou un entier de 1 à 29, n3 est zéro ou un entier de 1 à 20, n5 est zéro ou un entier de 1 à 17, n7 est zéro ou un entier de 1 à 14 et n9 est zéro ou un entier de 1 à 11; et chacun de n2, n4, n6 et n8 est indépendamment zéro ou 1 ; et Y² est CH₃, CO₂H, SH, S(O)₂OH, P(O)₂OH, OP(O)₂OH, OH, NH₂; où chaque carbone de Y¹-Y² peut être indépendamment substitué par des halogènes et des substituants aliphatiques,
R³ est sélectionné parmi l'acide phosphatidique (PO₂-OH), des dérivés de l'acide phosphatidique et des bioesters de l'acide phosphatidique et leurs dérivés.

20. Système d'apport de médicament à base de lipide selon la revendication 19, où R² est un groupe aliphatique d'une longueur d'au moins 7 atomes de carbone.

21. Système d'apport de médicament à base de lipide selon la revendication 6, où R² est un groupe de formule Y¹Y².

22. Système d'apport de médicament à base de lipide selon les revendications 1 et 21, où le système est sous la forme de liposomes.

23. Système d'apport de médicament à base de lipide selon l'une quelconque des revendications 1 à 5 et 19 à 22, où la substance médicamenteuse est une substance thérapeutiquement et/ou prophylactiquement active sélectionnée parmi (i) des agents antitumoraux, (ii) des antibiotiques et des antifongiques, et (iii) des agents anti-inflammatoires.

24. Composition pharmaceutique comprenant le système d'apport de médicament selon l'une quelconque des revendications 1 à 5 et 19 à 23 et facultativement un véhicule pharmaceutiquement acceptable.

25. Système d'apport de médicament à base de lipide selon l'une quelconque des revendications 1 à 5, 19 à 23 pour utilisation en tant que médicament.

26. Utilisation d'un système d'apport de médicament selon l'une quelconque des revendications 1 à 5, 19 à 23 pour la préparation d'un médicament destiné au traitement de maladies et pathologies associées à une élévation localisée de l'activité de la phospholipase A2 extracellulaire dans le tissu mammalien.

27. Utilisation selon la revendication 26, où les maladies ou pathologies sont sélectionnées dans le groupe constitué par des pathologies inflammatoires et le cancer.

28. Utilisation selon la revendication 27, où le type de cancer est sélectionné dans le groupe constitué du cancer du cerveau, du cancer du poumon, du cancer du sein, du cancer du côlon, du cancer ovarien, de la leucémie, des lymphomes, des sarcomes et des carcinomes.

29. Utilisation selon l'une quelconque des revendications 26 et 27, où l'élévation de l'activité de la phospholipase A2 extracellulaire est d'au moins 25 % par comparaison au taux normal d'activité dans le tissu en question.
